# EUROPEAN PATENT SPECIFICATION

(11) **EP 2 918 259 B1**
(45) Date of publication and mention of the grant of the patent: **02.01.2019**
(21) Application number: 14000839.2
(22) Date of filing: 10.03.2014
(51) Int. Cl.: A61K 6/083, C07C 43/20, C07C 69/54

(54) **Dental composition based on condensed aromatic compounds**
Auf kondensierten aromatischen Verbindungen basierende dentale Zusammensetzung
Composition dentaire à base de composès aromatiques condensés

(43) Date of publication of application: 16.09.2015
(73) Proprietor: DENTSPLY DETREY GmbH, 78467 Konstanz (DE)
(72) Inventor: Prof. Dr. Klee, Joachim E., 78315 Radolfzell (DE); Dr. Weber, Christoph, 78464 Konstanz (DE); Dr. Maier, Maximilian, 40213 Düsseldorf (DE); Szillat, Florian, 40474 Düsseldorf (DE); Scheufler, Christian, 78234 Engen (DE); Prof. Dr. Ritter, Helmut, 42111 Wuppertal (DE)
(74) Representative: Hartz, Nikolai

(56) References cited:
- EP-A1- 2 230 286
- WO-A1-2014/025406

## Description

### Field of the Invention

The present invention relates to a dental composition. The present invention also relates to processes for the preparation of a dental composition. Furthermore, the present invention relates to a dental composition obtainable by a process of the invention. Finally, the present invention relates to the use of the composition of the present invention in a dental composition.

A dental composition of the present invention may be a dental adhesive composition, a dental pit and fissure sealer, a dental composite, and a root canal filling composition. A dental composition according to the present invention does not require the use of 2,2-bis[4-(2-hydroxy-3-methacryloyloxy propoxy) phenyl] propane (bis-GMA), bisphenol-A dimethacrylate, ethoxylated bis-GMA, or any other raw material based on bisphenol-A for providing superior properties including mechanical properties, refractive index, and viscosity even in the absence of solvents.

### Background of the invention

Conventional dental compositions often comprise polymerizable monomers. In view of reducing the polymerization shrinkage, conventional dental compositions frequently contain bis-GMA. However, bisphenol-A contained in bis-GMA and other polymerizable resins is problematic when leached from the polymerized dental composition. Bisphenol-A is an endocrine disruptor which may have adverse effects on the patient and the dentist alike. Therefore, the use of any bisphenol-A containing polymerizable resin such as bis-GMA in a dental composition is not preferred.

WO2014025406 discloses dental materials made from resin which include segments of the following Formula (IA) wherein w is 4.

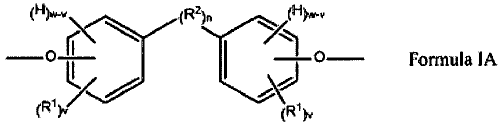

In formula (IA), R² may be present or absent. WO2014025406 observes that when R² is absent in the segment of Formula IA, either (i) a carbon atom of one phenylene ring is covalently attached to a carbon atom of the other phenylene ring (which occurs when w is 4) or (ii) the phenylene groups depicted in Formula IA join to form a fused ring system (which occurs when w is 3 and the phenylene groups are so fused). WO2014025406 discloses an embodiment of the segment of Formula IA in which n is 0, and w is 3 such that two phenylene groups have joined to form a naphtalene group as follows:

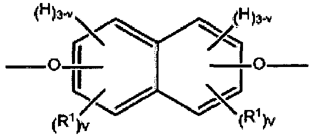

Given that w in Formula IA can only be 4, the above embodiment wherein w is 3, is explicitly excluded from the disclosure of WO2014025406. Moreover, an embodiment wherein R² is absent and a carbon atom of one phenylene ring is covalently attached to a carbon atom of the other phenylene ring according to (i), is not preferred since according to WO2014025406. Rather, according to WO2014025406 it is preferred that R² is present and is an organic groups containing less than 15 carbon atoms, which is reflected by the specific examples of WO2014025406.

### Summary of the Invention

It is the problem of the present invention to provide a dental composition which does not require the use of 2,2-bis[4-(2-hydroxy-3-methacryloyloxy propoxy) phenyl] propane (bis-GMA), bisphenol-A dimethacrylate, ethoxylated bis-GMA, or any other raw material based on bisphenol-A for providing superior properties including mechanical properties, refractive index, and viscosity even in the absence of solvents.

It is a further problem of the present invention to provide processes for the preparation of a dental composition whereby the composition provides superior properties including mechanical properties, refractive index, and viscosity even in the absence of solvents and which does not require the use of 2,2-bis[4-(2-hydroxy-3-methacryloyloxy propoxy) phenyl] propane (bis-GMA), bisphenol-A dimethacrylate, ethoxylated bis-GMA, or any other raw material based on bisphenol-A.

Furthermore, it is the problem of the present invention to provide a use of the composition which does not require the use of 2,2-bis[4-(2-hydroxy-3-methacryloyloxy propoxy) phenyl] propane (bis-GMA), bisphenol-A dimethacrylate, ethoxylated bis-GMA, or any other raw material based on bisphenol-A for providing superior properties including mechanical properties, refractive index, and viscosity even in the absence of solvents.

According to a first aspect, the present invention provides a dental composition comprising a polymerizable compound of the following formula (I), (II) and/or formula (III): 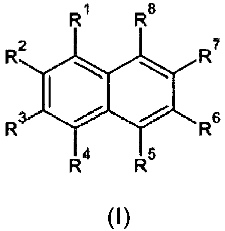 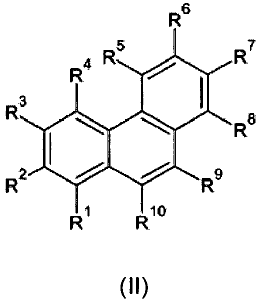 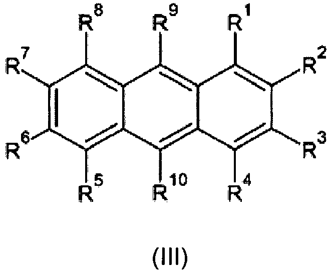 wherein
2 to 4 groups of R¹, R², R³, R⁴, R⁵, R⁶, R⁷, and R⁸, and, if present, R⁹, and R¹⁰, which are independent from each other, represent a group of the following formula (IV), (V), or (VI): 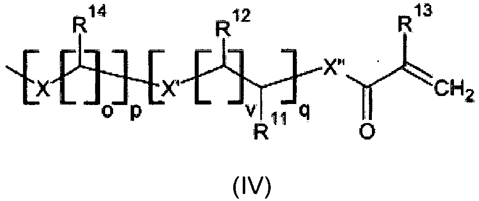 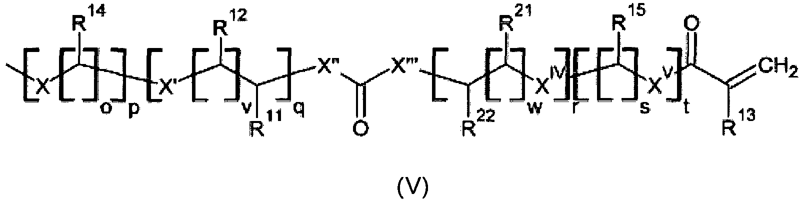 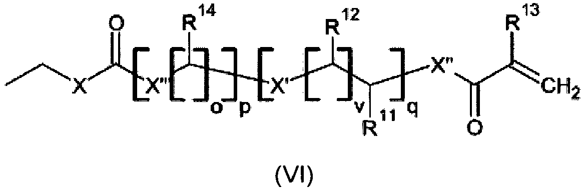 wherein
- X: is -NH-, an oxygen atom or a sulfur atom,
- X': is -NH-, an oxygen atom or a sulfur atom,
- X": is -NH-, an oxygen atom or a sulfur atom,
- X'": is -NH-, an oxygen atom or a sulfur atom,
- X^{IV}: is -NH-, an oxygen atom or a sulfur atom,
- X^{V}: is -NH-, an oxygen atom or a sulfur atom,
- R¹¹, R¹², R²¹ and R²²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- R¹⁴ and R¹⁵: which may be the same or different, independently represent a hydrogen atom, a hydroxyl group which may be substituted, or a straight chain or branched C₁₋₆ alkyl group,
- o and s: which may be the same or different, independently represent an integer of from 2 to 10,
- p and t: which may be the same or different, independently is an integer of from 0, 1 or 2;
- q and r: which may be the same or different, independently represent an integer of from 0 to 10, preferably 1 to 10;
- v and w: which may be the same or different, independently represent an integer of from 1 to 10
and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

Furthermore, according to a second aspect, the present invention provides a process for the preparation of a dental composition according to the first aspect, which process comprises the steps of

### (a) subjecting a reaction mixture comprising

(a1) one or more compounds of formula (VII), (VIII) and/or formula (IX): 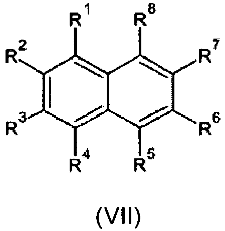 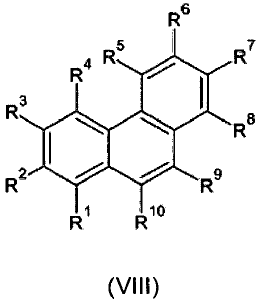 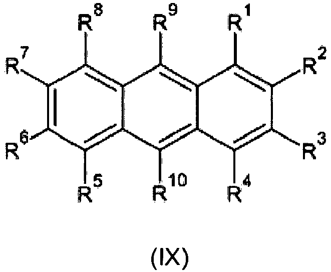 wherein
   2 to 4 groups of R¹, R², R³, R⁴, R⁵, R⁶, R⁷, and R⁸, and, if present, R⁹, and R¹⁰ are -XH groups wherein X is -NH-, an oxygen atom or a sulfur atom, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, and
(a2) epichlorohydrine, ethylene carbonate or a compound of the formula ZRZ', wherein Z and Z' independently are leaving groups and R is a straight chain or branched alkylene group,
   to an addition reaction for obtaining an addition product and
(b1) reacting the addition product of epichlorohydrine with a compound of the following formula (XI): 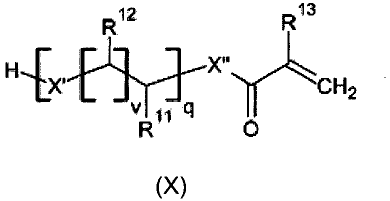 wherein
   - R¹¹ and R¹²: which may be the same or different, independently represent a hydrogen atom or a straight chain or branched C₁₋₆ alkyl group,
   - R¹³: represents a hydrogen atom or a straight chain or branched C₁₋₆ alkyl group which may be substituted by a hydroxyl group or carboxyl group,
   - X': is -NH-, an oxygen atom or a sulfur atom,
   - X": is -NH-, an oxygen atom or a sulfur atom, and
   - q: independently represents an integer of from 0 to 10, preferably 1 to 10,
   - v: independently represents an integer of from 1 to 10,
   for obtaining a polymerizable compound of formula (I), (II) or (III) of the present invention; or
(b2) reacting the addition product of ethylene carbonate with a compound of the following formula (XI): 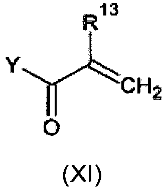 wherein Y is a leaving group and R¹³ represents a hydrogen atom or a straight chain or branched C₁₋₆ alkyl group, or wherein Y and R¹³ form together with the carbon atoms to which they are bound a cyclic lactone ring or carboxylic acid anhydride ring having 4 to 8 carbon atoms;
   for obtaining a polymerizable compound of formula (I), (II) or (III) of the present invention; or
(b3) reacting the addition product of the compound of the formula ZRZ', wherein Z, Z' and R are as defined above, with a compound of formula (XI) wherein Y is OH,
   for obtaining a polymerizable compound of formula (I), (II) or (III) of the present invention.

Furthermore, according to a third aspect, the present invention provides a process for the preparation of a dental composition according to the first aspect, comprising subjecting a reaction mixture comprising one or more compounds of formula (XII), (XIII) and/or formula (XIV): 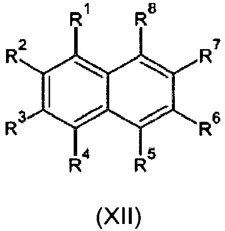 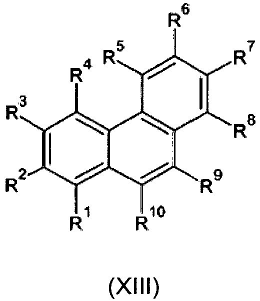 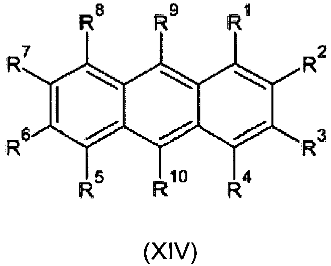 wherein
2 to 4 groups of R¹, R², R³, R⁴, R⁵, R⁶, R⁷, and R⁸, and, if present, R⁹, and R¹⁰ are independently a group of the following formula (XV): 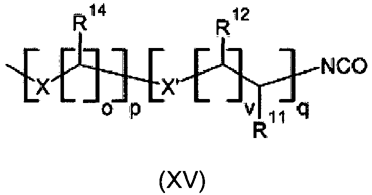 wherein
- X: is -NH-, an oxygen atom or a sulfur atom,
- X': is -NH-, an oxygen atom or a sulfur atom,
- R¹¹ and R¹²: which may be the same or different, independently represent a hydrogen atom or a straight chain or branched C₁₋₆ alkyl group,
- R¹⁴: which may be the same or different, independently represent a hydrogen atom, a hydroxyl group, or a straight chain or branched C₁₋₆ alkyl group,
- o: independently represent an integer of from 2 to 10,
- p: independently is an integer of from 0, 1 or 2;
- q: independently represent an integer of from 0 to 10, preferably 1 to 10;
- v: independently represents an integer of from 1 to 10,
and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, to an addition reaction with a compound of the following formula (XVI): 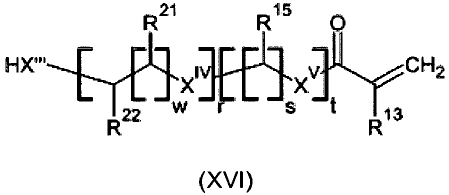 wherein
- X"': is -NH-, an oxygen atom or a sulfur atom,
- X^{IV}: is -NH-, an oxygen atom or a sulfur atom,
- X^{V}: is -NH-, an oxygen atom or a sulfur atom,
- R²¹ and R²²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- R¹⁵: which may be the same or different, independently represent a hydrogen atom, a hydroxyl group, or a straight chain or branched C₁₋₆ alkyl group,
- s: independently represent an integer of from 2 to 10,
- t: independently is an integer of from 0, 1 or 2;
- r: which may be the same or different, independently represent an integer of from 0 to 10, preferably 1 to 10;
- w: independently represents an integer of from 1 to 10,
for obtaining a polymerizable compound of formula (I), (II) or (III) as defined in the present invention.

Furthermore, according to a fourth aspect, the present invention provides a process for the preparation of a dental composition according to the first aspect, comprising subjecting a reaction mixture comprising one or more compounds of formula (XVII), (XVIII) and/or formula (XIX): 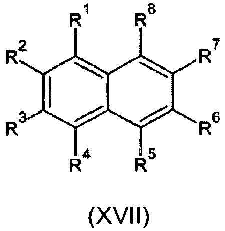 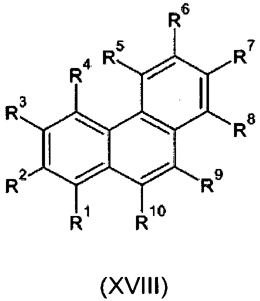 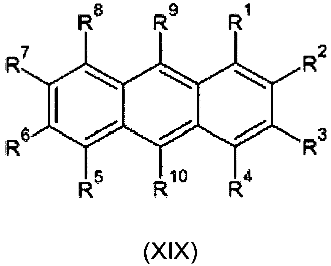 wherein
2 to 4 groups of R¹, R², R³, R⁴, R⁵, R⁶, R⁷, and R⁸, and, if present, R⁹, and R¹⁰ are isocyanatomethyl groups, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, to an addition reaction with a compound of the following formula (XX): 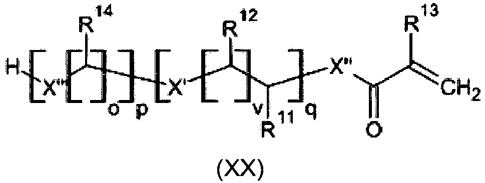 wherein
- X': is -NH-, an oxygen atom or a sulfur atom,
- X": is -NH-, an oxygen atom or a sulfur atom,
- X"': is -NH-, an oxygen atom or a sulfur atom,
- R¹¹ and R¹²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- R¹⁴: which may be the same or different, independently represent a hydrogen atom, a hydroxyl group, or a straight chain or branched C₁₋₆ alkyl group,
- o: which may be the same or different, independently represent an integer of from 2 to 10,
- p: which may be the same or different, independently is an integer of from 0, 1 or 2;
- q: which may be the same or different, independently represent an integer of from 0 to 10, preferably 1 to 10;
- v: independently represents an integer of from 1 to 10,
for obtaining a polymerizable compound of formula (I), (II) or (III) as defined in the present invention.

According to a specific embodiment of the sedond to fourth aspect, the process further comprises a step of reacting a polymerizable compound of formula (I), (II) or (III) as defined in the first aspect, which contains hydroxyl groups in any of R¹³, R¹⁴, and/or R¹⁵, with an isocyanate compound, a diisocyanate compound, isothiocyanate compound, a carboxylic acid halogenide compound, a cyclic carboxylic acid anhydride or a carboxylic acid compound for preparing a substituted hydroxyl group.

According to a fifth aspect, the present invention also provides a dental composition obtainable by any of the processes according to the present invention.

According to a sixth aspect, the present invention provides a use of a dental composition for the preparation of a dental composition as defined by the present invention.

### Detailed Description of the Preferred Embodiments

The present invention provides a dental composition. The dental composition comprises a polymerizable compound. The polymerizable compound may be a compound of the following general formula (I) which is a naphthalene derivative:

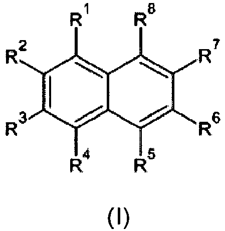

In a naphthalene derivative according to formula (I), any of R¹, R², R³, R⁴, R⁵, R⁶, R⁷, and R⁸ may be a hydrogen atom or the aromatic ring may be substituted at the respective position by a substituent group. The substituent groups may be groups having polymerizable substituent or saturated substituent groups which are not polymerizable. According to the present invention, 2 to 4 groups of R¹, R², R³, R⁴, R⁵, R⁶, R⁷, and R⁸ represent a polymerizable group and the remaining groups are hydrogen atoms or saturated groups which are not polymerizable. Accordingly, the compounds of formula (I) act as crosslinking monomer in a radical polymerization.

According to a first preferred embodiment, a compound of formula (I) contains 2 polymerizable substituents. According to a second preferred embodiment, a compound of formula (I) contains 3 or 4 polymerizable substituents.

In case the compound of formula (I) contains 2 polymerizable substituents, it is preferable that the substituents do not share the same annelated benzene ring. Accordingly, a first polymerizable substituent is present at R¹, R², R³, or R⁴, and a second polymerizable substituent is present at R⁵, R⁶, R⁷, or R⁸.

Preferably, the first and second polymerizable substituent form a non-straight angle which is preferably obtuse. The angle being defined by the formal valence bonds in formula (I).

Accordingly, the first and second polymerizable substituent are preferably not present at opposite positions across the aromatic ring system.

Specifically, when the first polymerizable substituent is present as R¹, then the second polymerizable substituent is preferably present as R⁶, R⁷, or R⁸. In this case it is less preferred that the second polymerizable substituent is present as R² or R³.

When the first polymerizable substituent is present as R² then the second polymerizable substituent preferably is present as R⁵, R⁷, or R⁸.

When the first polymerizable substituent is present as R³ then the second polymerizable substituent is preferably present as R⁵, R⁶, or R⁸.

When the first polymerizable substituent is present as R⁴, then the second polymerizable substituent is preferably present as R⁵, R⁶, or R⁷. In this case it is less preferred that the second polymerizable substituent is present as R² or R³.

When the first polymerizable substituent is present as R⁵, then the second polymerizable substituent is preferably present as R¹, R², or R³. In this case it is less preferred that the second polymerizable substituent is present as R⁶ or R⁷.

When the first polymerizable substituent is present as R⁶, then the second polymerizable substituent is preferably present as R¹, R³, or R⁴.

When the first polymerizable substituent is present as R⁷ then the second polymerizable substituent is preferably present as R¹, R², or R⁴.

When the first polymerizable substituent is present as R⁸, then the second polymerizable substituent is preferably present as R¹, R², or R³. In this case it is less preferred that the second polymerizable substituent is present as R⁶ or R⁷.

The remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

When the first polymerizable substituent is present as R⁷ then the second polymerizable substituent preferably present as R¹, R², or R⁴.

When a compound of formula (I) contains 3 or 4 polymerizable substituents, then depending on the first polymerizable substituent, the second, third, and optionally fourth substituent are preferably present at the positions considered preferred for the second substituent in the embodiment wherein a compound of formula (I) contains 2 polymerizable substituents above.

The remaining saturated substituent groups of R¹, R², R³, R⁴, R⁵, R⁶, R⁷, and R⁸, are not polymerizable. Specifically, the remaining groups independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups. According to a preferred embodiment, the remaining groups are hydrogen atoms.

Alternatively, the polymerizable compound may be a compound of the following general formula (II) which is a phenanthrene derivative:

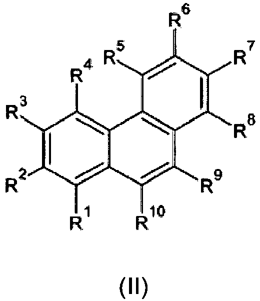

In a phenanthrene derivative according to formula (I), any of R¹, R², R³, R⁴, R⁵, R⁶, R⁷, R⁸, R⁹ and R¹⁰, may be a hydrogen atom or the aromatic ring may be substituted at the respective position by a substituent group. The substituent groups may be groups having polymerizable substituent or saturated substituent groups which are not polymerizable. According to the present invention, 2 to 4 groups of R¹, R², R³, R⁴, R⁵, R⁶, R⁷, R⁸, R⁹ and R¹⁰represent a polymerizable group and the remaining groups are hydrogen atoms or saturated groups which are not polymerizable. Accordingly, the compounds of formula (II) act as crosslinking monomer in a radical polymerization.

According to a first preferred embodiment, a compound of formula (II) contains 2 polymerizable substituents. According to a second preferred embodiment, a compound of formula (II) contains 3 or 4 polymerizable substituents.

In case the compound of formula (II) contains 2 polymerizable substituents, it is preferable that the substituents do not share the same annelated benzene ring. Accordingly, a first polymerizable substituent is present at R¹, R², R³, or R⁴, and a second polymerizable substituent is present at R⁵, R⁶, R⁷, or R⁸ or at R⁹ or R¹⁰.

Preferably, the first and second polymerizable substituent form a non-straight angle which is preferably obtuse. The angle being defined by the formal valence bonds in formula (II). Accordingly, the first and second polymerizable substituent are preferably not present at opposite positions across the aromatic ring system.

Specifically, when the first polymerizable substituent is present as R¹, then the second polymerizable substituent is preferably present as R⁵, R⁷, R⁸, and R⁹. In this case it is less preferred that the second polymerizable substituent is present as R², R³, R⁴, R⁶ and R¹⁰.

When the first polymerizable substituent is present as R² then the second polymerizable substituent preferably is present as R⁵, R⁶, R⁸, R⁹ and R¹⁰. In this case it is less preferred that the second polymerizable substituent is present as R¹, R³, or R⁴.

When the first polymerizable substituent is present as R³ then the second polymerizable substituent is preferably present as R⁶, R⁷, and R¹⁰. In this case it is less preferred that the second polymerizable substituent is present as R¹, R², or R⁴.

When the first polymerizable substituent is present as R⁴, then the second polymerizable substituent is preferably present as R⁷, R⁸, and R⁹. In this case it is less preferred that the second polymerizable substituent is present as R¹, R² or R³.

When the first polymerizable substituent is present as R⁵, then the second polymerizable substituent is preferably present as R¹, R², or R³. In this case it is less preferred that the second polymerizable substituent is present as R⁶, R⁷ or R⁸.

When the first polymerizable substituent is present as R⁶, then the second polymerizable substituent is preferably present as R², R³, or R⁹. In this case it is less preferred that the second polymerizable substituent is present as R⁵, R⁷ or R⁸.

When the first polymerizable substituent is present as R⁷ then the second polymerizable substituent is preferably present as R¹, R³, or R⁴. In this case it is less preferred that the second polymerizable substituent is present as R⁵, R⁶ or R⁸.

When the first polymerizable substituent is present as R⁸, then the second polymerizable substituent is preferably present as R¹, R², R⁴, or R¹⁰. In this case it is less preferred that the second polymerizable substituent is present as R⁶ or R⁷.

When the first polymerizable substituent is present as R⁹, then the second polymerizable substituent is preferably present as R¹, R², R⁶, R⁷, or R¹⁰. In this case it is less preferred that the second polymerizable substituent is present as R⁴ or R⁵.

When the first polymerizable substituent is present as R¹⁰, then the second polymerizable substituent is preferably present as R², R³, R⁵, R⁷, R⁸, or R⁹. In this case it is less preferred that the second polymerizable substituent is present as R⁴ or R⁵.

The remaining groups of R¹, R², R³, R⁴, R⁵, R⁶, R⁷, R⁸, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

When a compound of formula (I) contains 3 or 4 polymerizable substituents, then depending on the first polymerizable substituent, the second, third, and optionally fourth substituent are preferably present at the positions considered preferred for the second substituent in the embodiment wherein a compound of formula (II) contains 2 polymerizable substituents above.

The remaining saturated substituent groups of R¹, R², R³, R⁴, R⁵, R⁶, R⁷, and R⁸, are not polymerizable. Specifically, the remaining groups independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups. According to a first preferred embodiment, the remaining groups are hydrogen atoms. According to a second preferred embodiment, the remaining groups comprise halogen atoms. Bromine and iodine atoms are preferable as halogen atoms in view of radioopacity and refractive index.

Alternatively, the polymerizable compound may be a compound of the following general formula (III) which is an anthracene derivative:

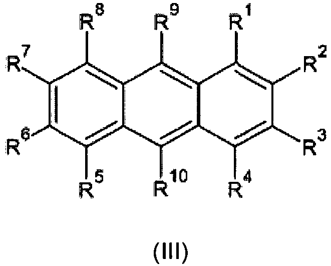

In an anthracene derivative according to formula (III), any of R¹, R², R³, R⁴, R⁵, R⁶, R⁷, R⁸, R⁹ and R¹⁰ may be a hydrogen atom or the aromatic ring may be substituted at the respective position by a substituent group. The substituent groups may be groups having polymerizable substituent or saturated substituent groups which are not polymerizable. According to the present invention, 2 to 4 groups of R¹, R², R³, R⁴, R⁵, R⁶, R⁷, R⁸, R⁹ and R¹⁰ represent a polymerizable group and the remaining groups are hydrogen atoms or saturated groups which are not polymerizable. Accordingly, the compounds of formula (III) act as crosslinking monomer in a radical polymerization.

According to a first preferred embodiment, a compound of formula (III) contains 2 polymerizable substituents. According to a second preferred embodiment, a compound of formula (III) contains 3 or 4 polymerizable substituents.

In case the compound of formula (III) contains 2 polymerizable substituents, it is preferable that the substituents do not share the same annelated benzene ring. Accordingly, a first polymerizable substituent is present at R¹, R², R³, or R⁴, and a second polymerizable substituent is present at R⁵, R⁶, R⁷, or R⁸ or at R⁹ or R¹⁰.

Preferably, the first and second polymerizable substituent form a non-straight angle which is preferably obtuse. The angle being defined by the formal valence bonds in formula (III). Accordingly, the first and second polymerizable substituent are preferably not present at opposite positions across the aromatic ring system.

Specifically, when the first polymerizable substituent is present as R¹, then the second polymerizable substituent is preferably present as R⁶ and R⁷. In this case it is less preferred that the second polymerizable substituent is present as R² and R³.

When the first polymerizable substituent is present as R² then the second polymerizable substituent preferably is present as R⁵, R⁷, R⁸, R⁹ and R¹⁰. In this case it is less preferred that the second polymerizable substituent is present as R¹, R³, or R⁴.

When the first polymerizable substituent is present as R³ then the second polymerizable substituent is preferably present as R⁵, R⁶, R⁸, R⁹, and R¹⁰. In this case it is less preferred that the second polymerizable substituent is present as R¹, R², or R⁴.

When the first polymerizable substituent is present as R⁴, then the second polymerizable substituent is preferably present as R⁶ and R⁷. In this case it is less preferred that the second polymerizable substituent is present as R² and R³.

When the first polymerizable substituent is present as R⁵, then the second polymerizable substituent is preferably present as R² and R³. In this case it is less preferred that the second polymerizable substituent is present as R² and R³.

When the first polymerizable substituent is present as R⁶, then the second polymerizable substituent is preferably present as R¹, R³, R⁴, R⁹, and R¹⁰. In this case it is less preferred that the second polymerizable substituent is present as R⁵, R⁷, or R⁸.

When the first polymerizable substituent is present as R⁷ then the second polymerizable substituent is preferably present as R¹, R², R⁴, R⁹, and R¹⁰. In this case it is less preferred that the second polymerizable substituent is present as R⁵, R⁶, or R⁸.

When the first polymerizable substituent is present as R⁸, then the second polymerizable substituent is preferably present as R² and R³. In this case it is less preferred that the second polymerizable substituent is present as R⁶ and R⁷.

When the first polymerizable substituent is present as R⁹, then the second polymerizable substituent is preferably present as R², R³, R⁶, or R⁷. In this case it is less preferred that the second polymerizable substituent is present as R¹⁰.

When the first polymerizable substituent is present as R¹⁰, then the second polymerizable substituent is preferably present as R², R³, R⁶, or R⁷. In this case it is less preferred that the second polymerizable substituent is present as R¹⁰.

The remaining groups of R¹, R², R³, R⁴, R⁵, R⁶, R⁷, R⁸, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

When a compound of formula (III) contains 3 or 4 polymerizable substituents, then depending on the first polymerizable substituent, the second, third, and optionally fourth substituent are preferably present at the positions considered preferred for the second substituent in the embodiment wherein a compound of formula (III) contains 2 polymerizable substituents above.

The remaining saturated substituent groups of R¹, R², R³, R⁴, R⁵, R⁶, R⁷, and R⁸, are not polymerizable. Specifically, the remaining groups independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups. According to a first preferred embodiment, the remaining groups are hydrogen atoms.

The polymerizable substituents are independent from each other, and represent a group of the formula (IV), (V), or (VI).

Formula (IV) is as follows: 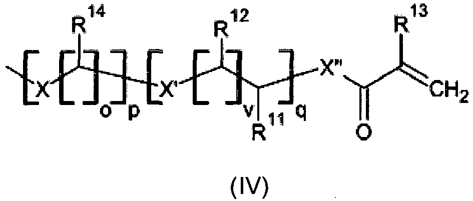 In formula (IV), units p may be present or absent. Accordingly, p may be 0 or an integer of 1 or 2. In case unit p is absent, then X' is directly bonded to the group of Formula (I), (II), or (III). In case unit p is present, then X is directly bonded to the group of Formula (I), (II), or (III). In formula (IV), X may be -NH-, an oxygen atom or a sulfur atom. Preferably, X is an oxygen atom. In formula (IV), X' may be -NH-, an oxygen atom or a sulfur atom. Preferably, X" is an oxygen atom. Units p contains repeating units o, whereby o is an integer of from 2 to 10. Repeating units o contain groups R¹⁴ which may be the same or different, and independently represent a hydrogen atom, a hydroxyl group, or a straight chain or branched C₁₋₆ alkyl group. Preferably, the R¹⁴ are independently a hydrogen atom or a hydroxyl group. According to a preferred embodiment, all R¹⁴ are hydrogen atoms so that unit o is a straight chain C₂₋₁₀ alkylene group, more preferably a C₂₋₈ alkylene group, in particular ethylene, propylene, butylene, pentylene, hexylene, heptylene, or octylene. In a specific embodiment o is 3, and the R¹⁴ are a hydrogen atom, a hydroxyl group and a hydrogen atom as obtainable by nucleophilic substitution of epichlorohydrine.

In formula (IV), q represents an integer of from 0 to 10, preferably 1 to 10. Units q contain group R¹¹ and units v including groups R¹².

The R¹¹ independently represent a hydrogen atom or a straight chain or branched C₁₋₆ alkyl group. Preferably, R¹¹ represent a hydrogen atom or a methyl group. The R¹¹ may be the same or different when q is greater than 1.

v represents an integer of from 1 to 10. Units v may be the same or different when q is greater than 1. Moreover, groups R¹² may be the same or different when v is greater than 1. Groups R¹² independently represent a hydrogen atom or a straight chain or branched C₁₋₆ alkyl group. Preferably, R¹² represent a hydrogen atom or a methyl group.

In formula (IV), a polymerizable group is attached to X". X" is -NH-, an oxygen atom or a sulfur atom. Preferably, X" is -NH- or an oxygen atom. In the polymerizable group, R¹³ represents a hydrogen atom or a straight chain or branched C₁₋₆ alkyl group which may be substituted by a hydroxyl group or carboxyl group. Preferably, R¹³ represents a hydrogen atom or a methyl group.

Particularly preferred polymerizable substituents of the formula (IV) wherein p is 0 are as follows:

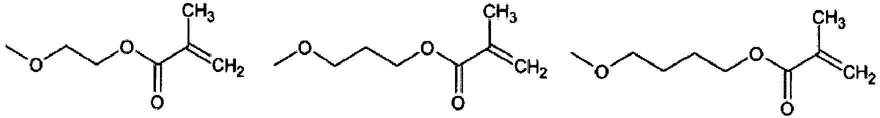

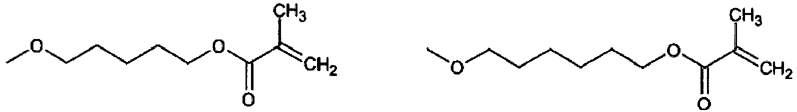

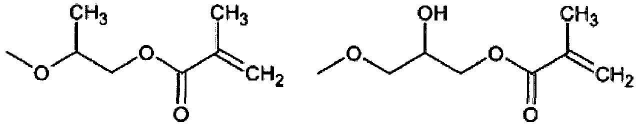

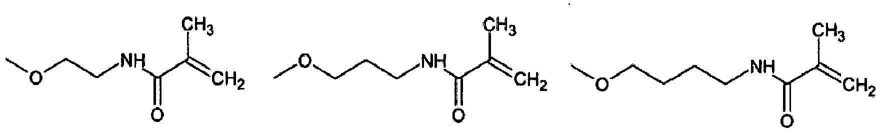

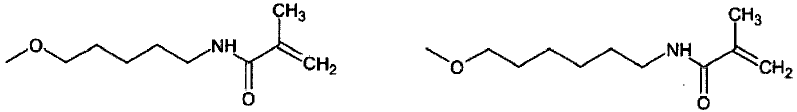

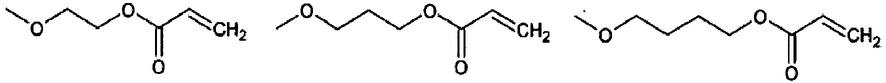

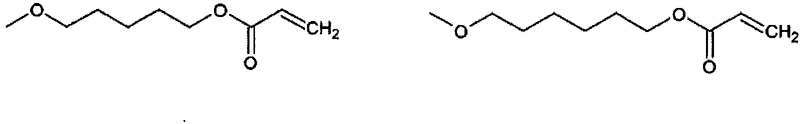

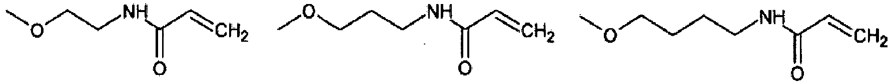

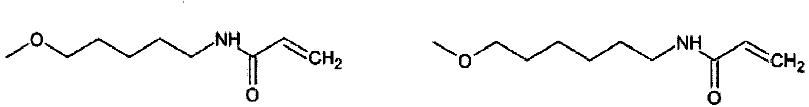

Particularly preferred polymerizable substituents of the formula (IV) wherein p is 1 are as follows:

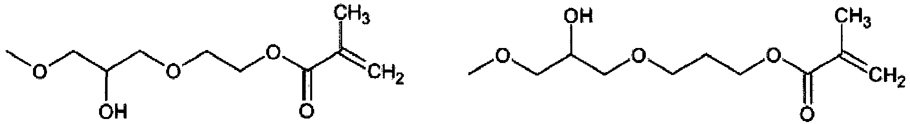

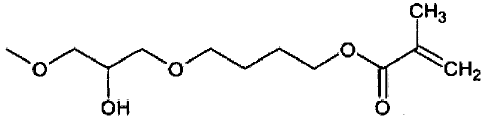

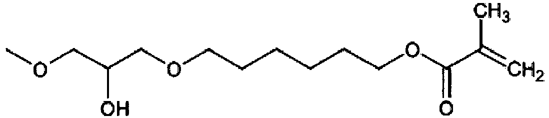

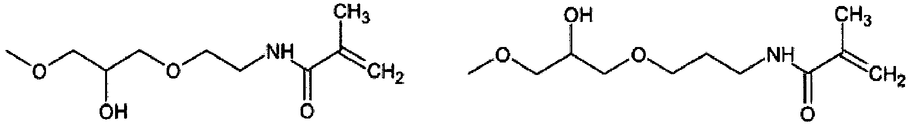

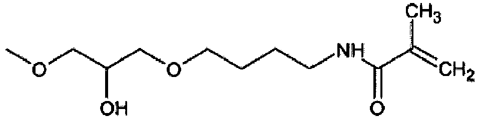

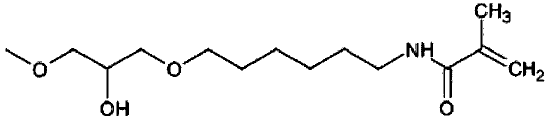

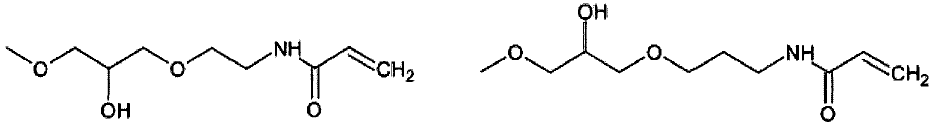

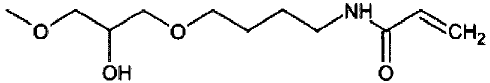

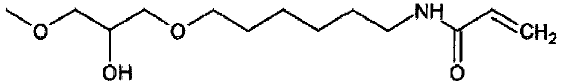

According to a preferred embodiment, a compound of formula (I), (II), or (III) contains the same 2 to 4 polymerizable substituents, which are groups of the formula (IV).

Formula (V) is as follows: 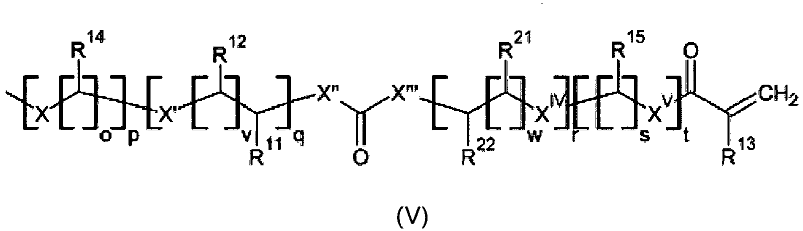 In formula (V), units p may be present or absent. Accordingly, p may be 0 or an integer of 1 or 2. In case unit p is absent, then X' is directly bonded to the group of Formula (I), (II), or (III). In case unit p is present, then X is directly bonded to the group of Formula (I), (II), or (III). In formula (V), X may be -NH-, an oxygen atom or a sulfur atom. Preferably, X is an oxygen atom. In formula (V), X' may be -NH-, an oxygen atom or a sulfur atom. Preferably, X" is an oxygen atom. Units p contains repeating units o, whereby o is an integer of from 2 to 10. Repeating units o contain groups R¹⁴ which may be the same or different, and independently represent a hydrogen atom, a hydroxyl group, or a straight chain or branched C₁₋₆ alkyl group. Preferably, the R¹⁴ are independently a hydrogen atom or a hydroxyl group. According to a preferred embodiment, all R¹⁴ are hydrogen atoms so that unit o is a straight chain C₂₋₁₀ alkylene group, more preferably a C₂₋₈ alkylene group, in particular ethylene, propylene, butylene, pentylene, hexylene, heptylene, or octylene. In a specific embodiment o is 3, and the R¹⁴ are a hydrogen atom, a hydroxyl group and a hydrogen atom as obtainable by nucleophilic substitution of epichlorohydrine.

In formula (V), q represents an integer of from 0 to 10, preferably 1 to 10. Units q contain group R¹¹ and units v including groups R¹².

The R¹¹ independently represent a hydrogen atom or a straight chain or branched C₁₋₆ alkyl group. Preferably, R¹¹ represent a hydrogen atom or a methyl group. The R¹¹ may be the same or different when q is greater than 1.

v represents an integer of from 1 to 10. Units v may be the same or different when q is greater than 1. Moreover, groups R¹² may be the same or different when v is greater than 1. Groups R¹² independently represent a hydrogen atom or a straight chain or branched C₁₋₆ alkyl group. Preferably, R¹² represent a hydrogen atom or a methyl group.

In formula (V), r represents an integer of from 0 to 10, preferably 1 to 10. Units r contain group R²² and units w including groups R²¹.

The R²² independently represent a hydrogen atom or a straight chain or branched C₁₋₆ alkyl group. Preferably, the R²² represent a hydrogen atom or a methyl group. The R²² may be the same or different when r is greater than 1.

w represents an integer of from 1 to 10. Units w may be the same or different when r is greater than 1. Moreover, groups R²¹ may be the same or different when w is greater than 1. Groups R²¹ independently represent a hydrogen atom or a straight chain or branched C₁₋₆ alkyl group. Preferably, R²¹ represents a hydrogen atom or a methyl group.

Units t contain repeating units s, whereby s is an integer of from 2 to 10. Repeating units s contain groups R¹⁵ which may be the same or different, and independently represent a hydrogen atom, a hydroxyl group, or a straight chain or branched C₁₋₆ alkyl group. Preferably, the R¹⁵ are independently a hydrogen atom or a hydroxyl group. According to a preferred embodiment, all R¹⁵ are hydrogen atoms so that unit s is a straight chain C₂₋₁₀ alkylene group, more preferably a C₂₋₈ alkylene group, in particular ethylene, propylene, butylene, pentylene, hexylene, heptylene, or octylene. In a specific embodiment s is 3, and the R¹⁵ are a hydrogen atom, a hydroxyl group and a hydrogen atom as obtainable by nucleophilic substitution of epichlorohydrine.

In formula (V), units t may be present or absent. Accordingly, t may be 0 or an integer of 1 or 2. In case unit t is absent, then the polymerizable group is directly bonded to X^{IV}. In case unit p is present, then the polymerizable group is directly bonded to X^{V}. In the polymerizable group, R¹³ represents a hydrogen atom or a straight chain or branched C₁₋₆ alkyl group which may be substituted by a hydroxyl group or carboxyl group. Preferably, R¹³ represents a hydrogen atom or a methyl group.

In formula (V), X may be -NH-, an oxygen atom or a sulfur atom. Preferably, X is an oxygen atom. In formula (V), X' may be -NH-, an oxygen atom or a sulfur atom. Preferably, X' is an oxygen atom. In formula (V), X" may be -NH-, an oxygen atom or a sulfur atom. Preferably, X" is an oxygen atom. In formula (V), X''' may be -NH-, an oxygen atom or a sulfur atom. Preferably, X''' is an oxygen atom. In formula (V), X^{IV} may be -NH-, an oxygen atom or a sulfur atom. Preferably, X^{IV} is an oxygen atom. In formula (V), X^{V} may be -NH-, an oxygen atom or a sulfur atom. Preferably, X^{V} is an oxygen atom.

In formula (V), the moiety -X"COX"'- is preferably selected from a urethane group and a urea group. In case of a urethane group, X" may be an oxygen atom and X''' may be an - NH-, or X" may be an -NH- and X''' may be an oxygen atom.

Particularly preferred polymerizable substituents of the formula (IV) wherein p is 0 are as follows:

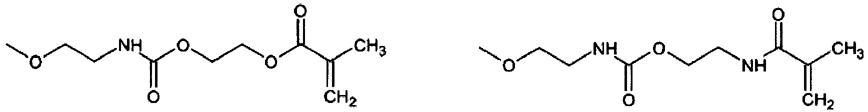

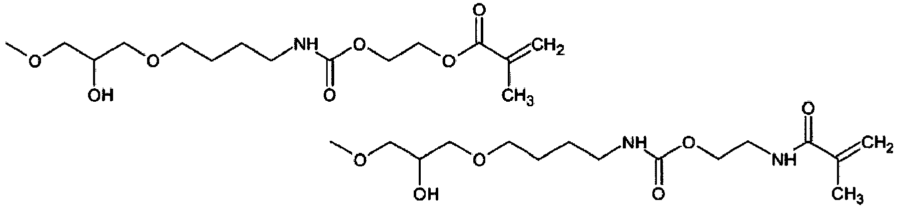

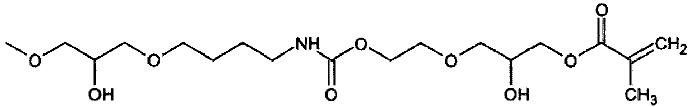

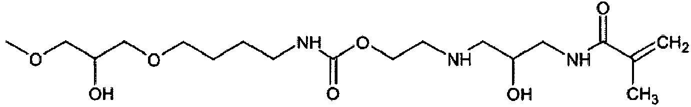

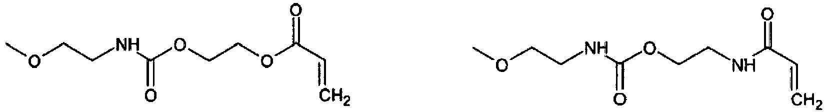

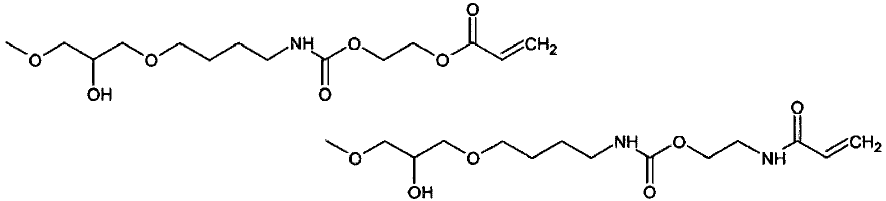

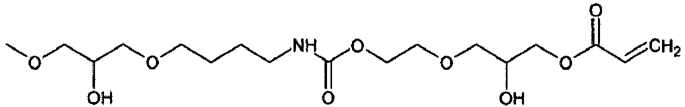

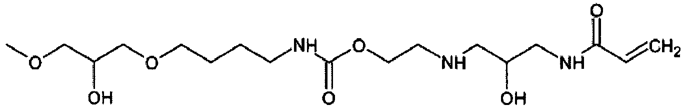

According to a preferred embodiment, a compound of formula (I), (II), or (III) contains the same 2 to 4 polymerizable substituents, which are groups of the formula (V).

Formula (VI) is as follows:

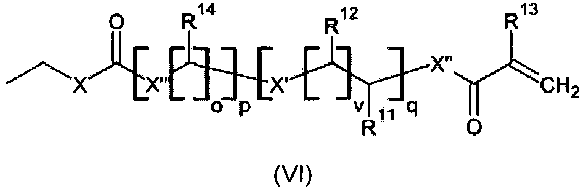

In formula (VI), units p may be present or absent. Accordingly, p may be 0 or an integer of 1 or 2. In case unit p is absent, then X' is directly bonded to the -X"CO group of formula (VI). In case unit p is present, then X''' is directly bonded to the -X"CO group of formula (VI).

In formula (VI), X may be -NH-, an oxygen atom or a sulfur atom. Preferably, X is -NH- or an oxygen atom. In formula (VI), X' may be -NH-, an oxygen atom or a sulfur atom. Preferably, X" is -NH- or an oxygen atom. In formula (VI), X''' may be -NH-, an oxygen atom or a sulfur atom. Preferably, X" is -NH- or an oxygen atom.

Units p contains repeating units o, whereby o is an integer of from 2 to 10. Repeating units o contain groups R¹⁴ which may be the same or different, and independently represent a hydrogen atom, a hydroxyl group, or a straight chain or branched C₁₋₆ alkyl group. Preferably, the R¹⁴ are independently a hydrogen atom or a hydroxyl group. According to a preferred embodiment, all R¹⁴ are hydrogen atoms so that unit o is a straight chain C₂₋₁₀ alkylene group, more preferably a C₂₋₈ alkylene group, in particular ethylene, propylene, butylene, pentylene, hexylene, heptylene, or octylene. In a specific embodiment o is 3, and the R¹⁴ are a hydrogen atom, a hydroxyl group and a hydrogen atom as obtainable by nucleophilic substitution of epichlorohydrine.

In formula (VI), q represents an integer of from 0 to 10, preferably 1 to 10. Units q contain group R¹¹ and units v including groups R¹².

The R¹¹ independently represent a hydrogen atom or a straight chain or branched C₁₋₆ alkyl group. Preferably, R¹¹ represent a hydrogen atom or a methyl group. The R¹¹ may be the same or different when q is greater than 1.

v represents an integer of from 1 to 10. Units v may be the same or different when q is greater than 1. Moreover, groups R¹² may be the same or different when v is greater than 1. Groups R¹² independently represent a hydrogen atom or a straight chain or branched C₁₋₆ alkyl group. Preferably, R¹² represent a hydrogen atom or a methyl group.

In formula (VI), a polymerizable group is attached to X". X" is -NH-, an oxygen atom or a sulfur atom. Preferably, X" is -NH- or an oxygen atom. In the polymerizable group, R¹³ represents a hydrogen atom or a straight chain or branched C₁₋₆ alkyl group which may be substituted by a hydroxyl group or carboxyl group. Preferably, R¹³ represents a hydrogen atom or a methyl group.

Particularly preferred polymerizable substituents of the formula (VI) wherein p is 0 are as follows:

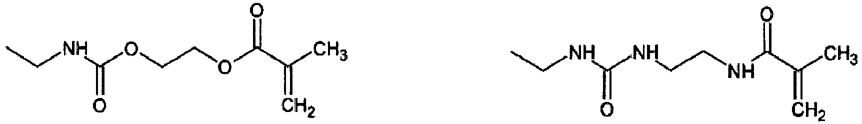

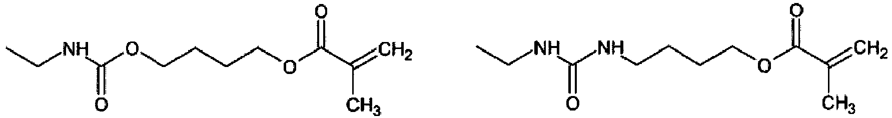

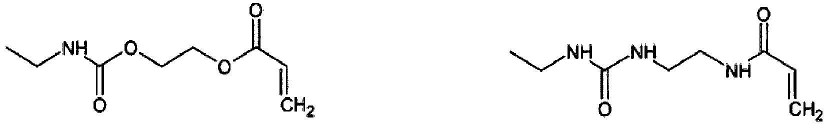

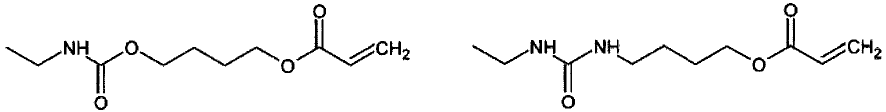

According to a preferred embodiment, a compound of formula (I), (II), or (III) contains the same 2 to 4 polymerizable substituents, which are groups of the formula (VI).

According to a preferred embodiment, the dental composition comprises a compound of formula (I).

Moreover, according to a further preferred embodiment, the dental composition comprises a compound, wherein 2 groups of R¹, R², R³, R⁴, R⁵, R⁶, R⁷, and R⁸, and, if present, R⁹, and R¹⁰, represent a group of the formula (IV). Preferably, the remaining groups of R¹, R², R³, R⁴, R⁵, R⁶, R⁷, and R⁸, and, if present, R⁹, and R¹⁰ represent a hydrogen atom.

According to a further preferred embodiment, the dental composition according to the present invention comprises a compound, wherein R² and R⁷, which may be the same or different, represent a group of formula (IV). Preferably, R¹, R³, R⁴, R⁵, R⁶ and R⁸ represent a hydrogen atom in this embodiment.

According to an alternative preferred embodiment, the dental composition comprises a compound of formula (II).

A preferred specific compound for use in the present invention is selected from (naphthalene-2,7-diylbis(oxy))bis(hexane-6, 1-diyl)bis(2-methacrylate), naphthalene-2,7-diylbis(oxy))bis(propane-3,1-diyl)bis(2-methacrylate), and (naphthalene-2,7-diylbis(oxy))bis(hydroxypropane-3,1-diyl)bis(2-methacrylate).

Preferably, the dental composition further comprises a filler and/or a solvent. Preferably, the dental composition further comprises a polymerization initiator system. The dental composition of the present invention may be a dental adhesive composition, a dental pit and fissure sealer, a dental composite, and a root canal filling composition. A dental composite may be a flowable dental composite, a universal dental composite, or packable dental composite. The dental composition is preferably selected from a dental adhesive composition, a dental pit and fissure sealer, a dental composite, and a root canal filling composition.

The dental composite of the present invention may comprise a particulate filler. A particulate filler is a powdered metal oxide or hydroxide, mineral silicate, or ion leachable glass or ceramic, or mixtures thereof. Examples of particulate fillers may be selected from fillers currently used in dental restorative compositions.

The particulate filler may have a unimodal or polymodal (e.g., bimodal) particle size distribution. The particulate filler can be an inorganic material. It can also be a crosslinked organic material that is insoluble in the polymerizable resin, and is optionally filled with inorganic filler. The particulate filler can be radiopaque, radiolucent or non-radiopaque.

Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable particulate organic filler particles include filled or unfilled pulverized polycarbonates or polyepoxides.

Preferably the surface of the filler particles is treated with a coupling agent in order to enhance the bond between the particulate filler and the matrix. The use of suitable coupling agents include gamma-methacryloxypropyltrimethoxysilane, gamma-mercaptopropyltriethoxysilane and gamma-aminopropyltrimethoxysilane.

The particulate filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus.

A dental composition of the present invention may contain nano-scale particles. As the nano-scale particles in the present invention, any known nano-scale particles used in dental compositions may be used without any limitation. Preferable examples of the nano-scale particles include particles of inorganic oxides such as silica, alumina, titania, zirconia, particles of composite oxides of any of these oxides, and particles of calcium phosphate, hydroxyapatite, yttrium fluoride and ytterbium fluoride. Preferably, the nano-scale particles are particles of silica, alumina, titania, prepared by flame pyrolysis.

The average particle size of the nano-scale particles is preferable 1 to 50 nm, and more preferably 3 to 40 nm. The average particle size of the nano-scale particles can be measured by taking electron micrographs of these nano-scale particles and calculating the average value of the diameters of the 100 randomly-selected nano-scale particles. It is desirable that the inorganic nano-scale particles be subjected previously to surface treatment with a surface treating agent to improve the affinity between the inorganic filler and the polymerizable composition of the present invention, and to increase the chemical bonding between the inorganic filler and the polymerizable composition so as to enhance the mechanical strength of the cured product.

The total amount of the particulate filler is preferably 0.1 to 500 parts by weight per 100 parts by weight of the polymerizable composition, more preferably 75 to 350 parts by weight, and particularly preferably 100 to 300 parts by weight. The amount of the nano-scale particles is preferably 0.1 to 50 parts by weight per 100 parts by weight of the polymerizable composition, more preferably 1 to 40 parts by weight, and particularly preferably 3 to 30 parts by weight.

Moreover, the polymerizable compound contained in the dental composition according to the present invention has a refractive index of from 1.500 to 1.540.

The dental composition of the present invention may comprise a solvent. A suitable solvent may be selected from water, alcohols, ketones, and ethers. Suitable alcohols may be selected from ethanol, iso-propanol, tert.-butanol and n-butanol. Suitable ketones may be selected from acetone and methyl ethyl ketone. Suitable ethers may be selected form diethyl ether and tetrahydrofurane. The solvent may be a mixture of two or more solvents.

The dental composite of the present invention may contain further polymerizable monomers other than the polymerizable compound of formula (I), (II), and (III). The polymerizable monomers may be mono-, bi-, tri- or polyfunctional monomers. The polymerizable monomers may be selected from methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol (meth)acrylamide, N-hydroxyethyl (meth)acrylamide, N-(dihydroxyethyl)(meth)acrylamide, (meth)acryloyloxydodecylpyridinium bromide, (meth)acryloyloxydodecylpyridinium chloride, (meth)acryloyloxyhexadeylpyridinium chloride, (meth)acryloyloxydecylammonium chloride, ethylene glycol di(meth)acrylate, triethylene glycol di(meth)acrylate, propylene glycol di(meth)acrylate, neopentyl glycol di(meth)acylate, 1,6-hexanediol di(meth)acrylate, 1,10-decanediol di(meth)acrylate, bisphenol A diglycidyl (meth)acrylate (2,2-bis[4-[3-(meth)acryloyloxy-2-hydroxypropoxy]phenyl]propane, commonly known as "BisGMA"), 2,2-bis[4-(meth)acryloyloxyethoxyphenyl]propane, 2,2-bis[4-(meth)acryloyloxyolyothoxyphenyl]propane, 2,2-bis[4-[3-((meth)acryloyloxy-2-hydroxypropoxy]phenyl]propane, 1,2-bis[3-(meth)acryloyloxy-2-hydroxypropoxylethane, pentaerythritol di(meth)acrylate, [2,2,4-trimethylhexamethylenebis(2-carbamoyloxyethyl)] dimethacrylate (commonly known as "UDMA trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarbonyloxy)propane-1,3-diol]tetramethacrylate, and 1,7-diacryloyloxy-2,2,6,6-tetraacryloyloxymethyl-4-oxyheptane.

The total amount of the polymerizable monomers contained in the dental composition besides the polymerizable compound of formula (I), (II), and (III) is preferably in the range of from 1 part to 100 part by weight per 100 part by weight of the polymerizable compound of formula (I), (II), and (III).

The dental composition of the present invention preferably contains a polymerization initiator. The type of the polymerization initiator is not particularly limited and can be selected from polymerization initiators commonly used in the dental field. Particularly, photopolymerization initiators and chemical polymerization initiators may b used alone, or two or more of them may be used in combination.

Examples of suitable photopolymerization initiators include alpha-diketones or (bis)acylphosphine oxides.

Examples of the alpha-diketones used as the photopolymerization initiator include camphorquinone, 9,10-phenanthrenequinone, 2,3-pentadione, 2,3-octadione, 4,4'-oxybenzyl, and acenaphthenequinone. Camphorquinone having the maximum absorption wavelength in the visible light range is preferred.

Examples of the acylphosphine oxides include 2,4,6-trimethylbenzoylmethoxyphenylphosphine oxide, 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylethoxyphenylphosphine oxide, 2,3,5,6-tetramethylbenzoyldiphenylphosphine oxide, and benzoyl di-(2,6-dimethylphenyl)phosphonate.

Examples of the bisacylphosphine oxides include bis-(2,6-dichlorobenzoyl)phenylphosphine oxide, bis-(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, bis-(2,6-dimethoxybenzoyl)phenylphosphine oxide, bis-(2,6-dimethoxybenzoyl)-2,4,4-trimethylpentylphosphine oxide, bis-(2,6-dimethoxybenzoyl)-2,5-dimethylphenylphosphine oxide, bis-(2,4,6-trimethylbenzoyl)phenylphosphine oxide, and (2,5,6-trimethylbenzoyl)-2,4,4-trimethylpentylphosphine oxide.

A chemical polymerization initiator may be an organic peroxide selected from ketone peroxide, hydroperoxide, diacyl peroxide, dialkyl peroxide, peroxyketal, peroxyester, and peroxydicarbonate.

A ketone peroxide may be selected from methyl ethyl ketone peroxide, methyl isobutyl ketone peroxide, methylcyclohexanone peroxide, and cyclohexanone peroxide.

A hydroperoxide may selected from 2,5-dimethylhexane-2,5-dihydroperoxide, diisopropylbenzene hydroperoxide, cumene hydroperoxide, and t-butyl hydroperoxide.

A diacyl peroxide may be selected from acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

A dialkyl peroxide may be selected from di-t-butyl peroxide, dicumyl peroxide, t-butyleumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

A peroxyketal may be selected from 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

A peroxyester may be selected form t-butvlperoxy acetate, t-butylperoxy-2 -ethyl hexanoate, alpha-cumyl peroxyneodecanoate, t-butyl peroxyneodecanoate, t-butyl peroxypivarate, 2,2,4-trimethylpentylperoxy-2-ethyl hexanoate, t-amylperoxy-2-ethyl hexanoate, di-t-butylperoxy isophthalate, di-t-butylperoxy hexahydroterephthalate, t-butylperoxy-3,3,5-trimethyl hexanoate, t-butylperoxy benzoate, and t-butylperoxymaleic acid.

A peroxydicarbonate maybe selected from di-3-methoxy peroxydicarbonate, di-2-ethylhexyl peroxydicarbonate, bis(4-t-butyleyelohexyl)peroxydicarbonate, diisopropyl peroxydicarbonate, di-n-propyl peroxydicarbonate, di-2-ethoxyethyl peroxydicarbonate, and diallyl peroxydicarbonate.

Benzoyl peroxide is preferred.

The amount of the polymerization initiator to be added in the present invention is not particularly limited. Preferably, 0.01 to 10 parts by weight of the polymerization initiator per 100 parts by weight of the polymerizable composition may be used. When the amount of the polymerization initiator is less than 0.01 part by weight, polymerization may not proceed sufficiently and thereby mechanical strength may be reduced. Therefore, the amount is more preferably at least 0.1 part by weight. On the other hand, when the amount of the polymerization initiator exceeds 10 parts by weight, in the case where the polymerization initiator itself has low polymerization performance, sufficient mechanical strength may not be obtained and furthermore precipitation from the composition may occur.

The dental composition of the present invention may further contain a polymerization accelerator. Examples of the polymerization accelerator are amines and sulfinic acids and salts thereof.

Amines may be aliphatic amines or aromatic amines. Examples of aliphatic amines include primary aliphatic amines such as n-butylamine, secondary aliphatic amines such as diisopropylamine, and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine, tertiary aliphatic amines are preferred. Aromatic amines may be selected from N,N-di(2-hydroxyethyl)-p-toluidine, 4-N,N-dimethylaminobenzoic acid ethyl ester, N,N-dimethylaminobenzoic acid n-butoxyethyl ester, and 4-N,N-dimethylaminobenzophenone.

A sulfinic acid or salt thereof may be selected from sodium benzenesulfinate, sodium p-toluenesulfinate, and sodium 2,4,6-triisopropylbenzenesulfinate.

The amount of polymerization accelerator is not particularly limited. The amount may be selected from the range of from 0.001 to 5 parts by weight of polymerization accelerator per 100 parts by weight of the polymerizable composition.

The dental composition of the present invention may further contain a pH adjuster, an ultraviolet absorber, an antioxidant, a polymerization inhibitor, a colorant, an antibacterial agent, an X-ray contrast agent, a thickening agent, a fluorescent agent.

The dental composition of the present invention may further contain a fluorine ion sustained-releasable filler, such as sodium fluoride, calcium fluoride, fluoroaluminosilicate glass, or sodium monofluorophosphate.

The dental composition may contain an antimicrobial agent. The antimicrobial agent may be a surfactant having an antibacterial activity, such as 12-(meth)acryloyloxydodecylpyridinium bromide or cetylpyridinium chloride.

The present invention also provides processes for the preparation of a dental composition according to the invention.

According to a first process, a reaction mixture is subjected to an addition reaction for obtaining an addition product. The reaction mixture comprises one or more compounds of formula (VII), (VIII) and/or formula (IX): 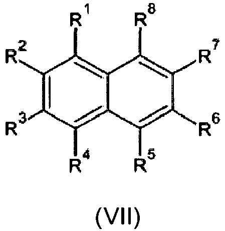 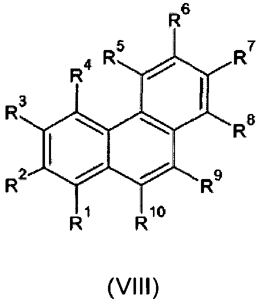 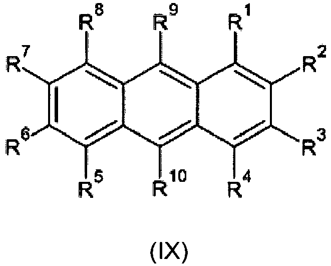 formula (VII), (VIII) and/or formula (IX), 2 to 4 groups of R¹, R², R³, R⁴, R⁵, R⁶, R⁷, and R⁸, and, if present, R⁹, and R¹⁰ are -XH groups wherein X is -NH-, an oxygen atom or a sulfur atom, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, and

The reaction mixture further comprises epichloro hydrine, ethylene carbonate or a compound of the formula ZRZ', wherein Z and Z' independently are leaving groups and R is a straight chain or branched alkylene group. The reaction product of epichlorohydrine may be reacted in an addition reaction with a polyoxyalkylene.

The reaction using epichlorohydrine may be carried out by mixing, preferably during heating, of components (VII), (VIII) and/or (IX) and epichloro hydrine in a reaction vessel and reacting the mixture in a single step in the presence of an addition catalyst for providing an addition product (Podkosielna B. et al.; Applied Suface Science 256 (2010) 2462-2467).

The reaction time is not particularly limited and may be selected in the range from 5 minutes to 8 hours. Preferably, the reaction time is selected in the range of from 10 minutes to 2 hours, more preferably, from 15 minutes to 1 hour.

The reaction temperature is not particularly limited and may be selected in the range from ambient temperature to the boiling temperature of the mixture. Preferably, the reaction temperature is selected in the range of from 50 °C to the 150 °C, more preferably from 70 °C to 130 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dimethyl sulfoxide, toluene, DMF, and ethyleneglycol monomethyl ether.

The reaction may be carried out in the presence of a catalyst. The catalyst may be a base. A suitable base may be selected from alkali metal hydroxides such as sodium hydroxide or potassium hydroxide.

The amount of the catalyst is not particularly limited and may be selected in a range of from 0.01 to 15 percent by weight, more preferably 0.1 to 3 percent by weight, based on the total weight of components (VII), (VIII), and (IX) present in the reaction mixture.

The obtained addition product may be soluble in organic solvents such as chloroform, DMF and THF and in reactive diluents such as triethyleneglycol dimethacrylate.

Subsequently, the process further comprises reacting the epichlorohydrine addition product with a compound of the following formula (X): 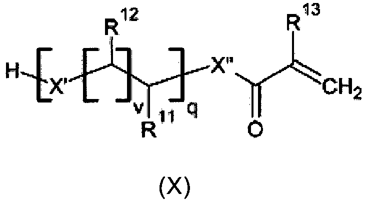 wherein
- R¹¹ and R¹²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- X': is -NH-, an oxygen atom or a sulfur atom,
- X": is -NH-, an oxygen atom or a sulfur atom, and
- q: independently represents an integer of from 0 to 10, preferably 1 to 10,
- v: independently represents an integer of from 1 to 10,
for obtaining a polymerizable compound of formula (I), (II) or (III) as defined above.

The reaction may be carried out by mixing, preferably during heating, of the addition product with epichlorohydrine and one or more a compounds of the formula (X) in a reaction vessel and reacting the mixture in a single step for providing a polymerizable compound of formula (I), (II) or (III) of the present invention. According to a preferred embodiment, when X' is - NH-, then q is at least 1.

The reaction time is not particularly limited and may be selected in the range from 30 minutes to 48 hours. Preferably, the reaction time is selected in the range of from 1 hours to 12 hours, more preferably, from 2 hours to 10 hours.

The reaction temperature is not particularly limited and may be selected in the range from ambient temperature to the boiling temperature of the mixture. Preferably, the reaction temperature is selected in the range of from 50 °C to the 150 °C, more preferably from 70 °C to 130 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dimethyl sulfoxide, toluene, DMF, and ethyleneglycol monomethyl ether. Preferably, the reaction is carried out in the absence of a solvent.

The reaction may be carried out in the presence of a catalyst. The catalyst may be a phase transfer catalyst. A suitable phase transfer catalyst may be selected from quaternary ammonium and phosphonium salts. Specifically, the phase transfer catalyst may be triethylbenzyl ammonium chloride, benzyltrimethyl ammonium chloride and hexadecyltributylphosphonium bromide. Preferably, triethylbenzyl ammonium chloride may be used.

The amount of the catalyst is not particularly limited and may be selected in a range of from 0.01 to 5 percent by weight, more preferably 0.1 to 3 percent by weight, based on the total weight of the addition product present in the reaction mixture.

When component (X) is present in the reaction mixture, the reaction may be carried out in the presence of an inhibitor. The inhibitor may be any conventionally known inhibitor which does not interfere with the desired reaction. The inhibitor may be selected from 2,6-di-tert-butyl-p-cresol (BHT), hydroquinone, hydroquinone monomethyl ether, tert.-butyl hydrochinone (TBHQ), TEMPO, and phenothiazin. Preferably, 2,6-di-tert-butyl-p-cresol may be used.

The amount of the inhibitor is not particularly limited and may be selected in a range of from 0.001 to 0.5 percent by weight, more preferably 0.01 to 0.3 percent by weight, based on the total weight of the addition product present in the reaction mixture.

The obtained methacrylate terminated macromer is may be soluble in organic solvents such as chloroform, DMF and THF and in reactive diluents such as triethyleneglycol dimethacrylate.

The reaction using ethylene carbonate may be carried out by mixing, preferably during heating, of components (VII), (VIII) and/or (IX) and ethylene carbonate in a reaction vessel and reacting the mixture in a single step in the presence of an addition catalyst for providing an addition product.

The reaction time is not particularly limited and may be selected in the range from 5 minutes to 8 hours. Preferably, the reaction time is selected in the range of from 20 minutes to 5 hours, more preferably, from 30 minutes 4 hour.

The reaction temperature is not particularly limited and may be selected in the range from ambient temperature to the boiling temperature of the mixture. Preferably, the reaction temperature is selected in the range of from 50 °C to the 250 °C, more preferably from 70 °C to 200 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dimethyl sulfoxide, toluene, DMF, and ethyleneglycol monomethyl ether.

The reaction may be carried out in the presence of a catalyst. The catalyst may be a base. A suitable base may be selected from alkali metal hydroxides such as sodium hydroxide or potassium hydroxide or alkali metal carbonates such as sodium carbonate and potassium carbonate.

The amount of the catalyst is not particularly limited and may be selected in a range of from 0.01 to 15 percent by weight, more preferably 0.1 to 10 percent by weight, based on the total weight of components (VII), (VIII), and (IX) present in the reaction mixture.

The obtained addition product may be soluble in organic solvents such as chloroform, DMF and THF and in reactive diluents such as triethyleneglycol dimethacrylate.

Subsequently, the process further comprises reacting the ethylene carbonate addition product with a compound of the following formula (XI): 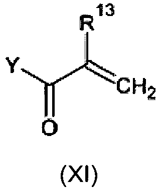 wherein Y is a leaving group and R¹³ represents a hydrogen atom or a straight chain or branched C₁₋₆ alkyl group, or wherein Y and R¹³ form together with the carbon atoms to which they are bound a cyclic lactone or carboxylic acid anhydride ring having 4 to 8 carbon atoms, for obtaining a polymerizable compound of formula (I), (II) or (III) of the present invention.

The reaction may be carried out by mixing, preferably during heating, of the addition product with ethylene carbonate and one or more a compounds of the formula (XI) in a reaction vessel and reacting the mixture in a single step for providing a polymerizable compound of formula (I), (II) or (III) of the present invention.

The reaction time is not particularly limited and may be selected in the range from 30 minutes to 48 hours. Preferably, the reaction time is selected in the range of from 1 hours to 30 hours, more preferably, from 2 hours to 24 hours.

The reaction temperature is not particularly limited and may be selected in the range from - 50 °C to the boiling temperature of the mixture. Preferably, the reaction temperature is selected in the range of from -30 °C to the 60 °C, more preferably from -10 °C to 40 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dichloromethane, chloroform, dimethyl sulfoxide, toluene, DMF, and ethyleneglycol monomethyl ether.

The reaction may be carried out in the presence of a catalyst. The catalyst may be a base. A suitable base may be selected from amines such as triethyl amine.

The amount of the catalyst is not particularly limited and may be selected in a range of from 0.01 to 5 percent by weight, more preferably 0.1 to 3 percent by weight, based on the total weight of the addition product present in the reaction mixture.

When component (XI) is present in the reaction mixture, the reaction may be carried out in the presence of an inhibitor. The inhibitor may be any conventionally known inhibitor which does not interfere with the desired reaction. The inhibitor may be selected from 2,6-di-tert-butyl-p-cresol (BHT), hydroquinone, hydroquinone monomethyl ether, tert.-butyl hydrochinone (TBHQ), TEMPO, and phenothiazin. Preferably, 2,6-di-tert-butyl-p-cresol may be used.

The amount of the inhibitor is not particularly limited and may be selected in a range of from 0.001 to 0.5 percent by weight, more preferably 0.01 to 0.3 percent by weight, based on the total weight of the addition product present in the reaction mixture.

The obtained methacrylate terminated macromer is may be soluble in organic solvents such as chloroform, DMF and THF and in reactive diluents such as triethyleneglycol dimethacrylate.

The reaction using a compound of the formula ZRZ', wherein Z and Z' independently are leaving groups and R is a straight chain or branched alkylene group, may be carried out by mixing, preferably during heating, of components (VII), (VIII) and/or (IX) and a compound of the formula ZRZ', in a reaction vessel and reacting the mixture in a single step in the presence of an addition catalyst for providing an addition product.

In the formula ZRZ', Z and Z' independently are leaving groups. The leaving groups may be selected from halogen atoms, a hydroxyl group and a tosyl group. A halogen atom may be a chlorine atom, a bromine atom and an iodine atom. A bromine atom is preferred. In the formula ZRZ', R is a straight chain or branched alkylene group. The alkylene group is preferably, a C₂₋₁₀ alkylene group, more preferably, a straight chain C₂₋₈ alkylene group. Examples of the alkylene group is ethylene, propylene, butylene, pentylene, hexylene, heptylene, and octylene. Preferably, a compound of the formula ZRZ' is a compound of the following formula Br(CH₂)ₙBr, wherein n is an integer of from 2 to 10, more preferably, from 2 to 8, still more preferably, 6.

The reaction time is not particularly limited and may be selected in the range from 1 hour to 36 hours. Preferably, the reaction time is selected in the range of from 2 hours to 30 hours, more preferably, from 5 hours to 24 hours.

The reaction temperature is not particularly limited and may be selected in the range from - 30 °C to the boiling temperature of the mixture. Preferably, the reaction temperature is selected in the range of from -10°C to the 100 °C, more preferably from 0 °C to 80 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as acetone, dichloromethane, dimethyl sulfoxide, toluene, DMF, and ethyleneglycol monomethyl ether.

The reaction may be carried out in the presence of a catalyst. The catalyst may be a base. A suitable base may be selected from alkali metal hydroxides such as sodium hydroxide or potassium hydroxide or alkali metal carbonates such as sodium carbonate and potassium carbonate.

The amount of the catalyst is not particularly limited and may be selected in a range of from 0.01 to 15 percent by weight, more preferably 0.1 to 10 percent by weight, based on the total weight of components (VII), (VIII), and (IX) present in the reaction mixture.

The obtained addition product may be soluble in organic solvents such as chloroform, DMF and THF and in reactive diluents such as triethyleneglycol dimethacrylate.

Subsequently, the process further comprises reacting the ethylene carbonate addition product with a compound of the following formula (XI): 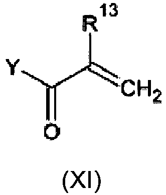 wherein Y is a leaving group and R¹³ represents a hydrogen atom or a straight chain or branched C₁₋₆ alkyl group, or wherein Y and R¹³ form together with the carbon atoms to which they are bound a cyclic lactone or carboxylic acid anhydride ring having 4 to 8 carbon atoms, for obtaining a polymerizable compound of formula (I), (II) or (III) of the present invention.

The reaction may be carried out by mixing, preferably during heating, of the addition product with a compound of the formula ZRZ', wherein Z and Z' independently are leaving groups and R is a straight chain or branched alkylene group, and one or more a compounds of the formula (XI) in a reaction vessel and reacting the mixture in a single step for providing a polymerizable compound of formula (I), (II) or (III) of the present invention.

The reaction time is not particularly limited and may be selected in the range from 30 minutes to 48 hours. Preferably, the reaction time is selected in the range of from 1 hours to 36 hours, more preferably, from 2 hours to 24 hours.

The reaction temperature is not particularly limited and may be selected in the range from - 50 °C to the boiling temperature of the mixture. Preferably, the reaction temperature is selected in the range of from 0°C to the 100 °C, more preferably from 10 °C to 90 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as ethyl acetate, acetone, dichloromethane, chloroform, dimethyl sulfoxide, toluene, DMF, and ethyleneglycol monomethyl ether.

The reaction may be carried out in the presence of a catalyst. The catalyst may be quaternary amine such as tertrabutylammonium iodide.

The amount of the catalyst is not particularly limited and may be selected in a range of from 0.01 to 5 percent by weight, more preferably 0.1 to 3 percent by weight, based on the total weight of the addition product present in the reaction mixture.

When component (XI) is present in the reaction mixture, the reaction may be carried out in the presence of an inhibitor. The inhibitor may be any conventionally known inhibitor which does not interfere with the desired reaction. The inhibitor may be selected from 2,6-di-tert-butyl-p-cresol (BHT), hydroquinone, hydroquinone monomethyl ether, tert.-butyl hydrochinone (TBHQ), TEMPO, and phenothiazin. Preferably, 2,6-di-tert-butyl-p-cresol may be used.

The amount of the inhibitor is not particularly limited and may be selected in a range of from 0.001 to 0.5 percent by weight, more preferably 0.01 to 0.3 percent by weight, based on the total weight of the addition product present in the reaction mixture.

The obtained methacrylate terminated macromer is may be soluble in organic solvents such as chloroform, DMF and THF and in reactive diluents such as triethyleneglycol dimethacrylate.

According to a second process of the present invention a reaction mixture is subjected to an addition reaction. The reaction mixture comprises one or more compounds of formula (XII), (XIII) and/or formula (XIV): 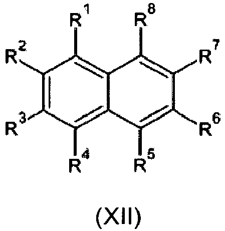 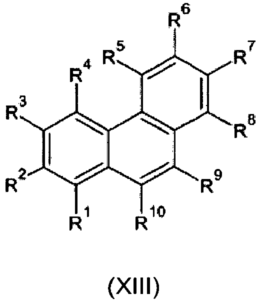 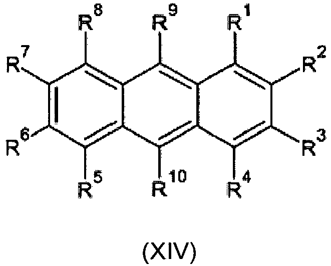 wherein
2 to 4 groups of R¹, R², R³, R⁴, R⁵, R⁶, R⁷, and R⁸, and, if present, R⁹, and R¹⁰ are independently a group of the following formula (XV): 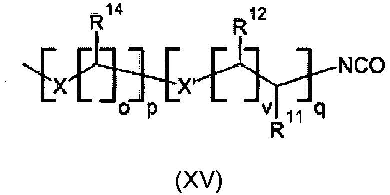 wherein
- X: is -NH-, an oxygen atom or a sulfur atom,
- X': is -NH-, an oxygen atom or a sulfur atom,
- R¹¹ and R¹²: which may be the same or different, independently represent a hydrogen atom or a straight chain or branched C₁₋₆ alkyl group,
- R¹⁴: which may be the same or different, independently represent a hydrogen atom, a hydroxyl group, or a straight chain or branched C₁₋₆ alkyl group,
- o: independently represent an integer of from 2 to 10,
- p: independently is an integer of from 0, 1 or 2;
- q: independently represent an integer of from 0 to 10, preferably 1 to 10;
- v: independently represents an integer of from 1 to 10,

and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

The addition reaction is carried out with a compound of the following formula (XVI): 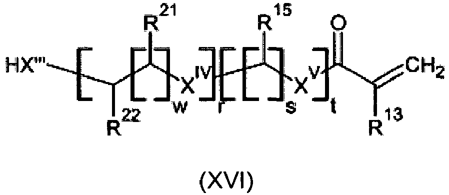 wherein
- X''': is -NH-, an oxygen atom or a sulfur atom,
- X^{IV}: is -NH-, an oxygen atom or a sulfur atom,
- X^{V}: is -NH-, an oxygen atom or a sulfur atom,
- R²¹ and R²²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- R¹⁵: which may be the same or different, independently represent a hydrogen atom, a hydroxyl group, or a straight chain or branched C₁₋₆ alkyl group,
- s: independently represent an integer of from 2 to 10,
- t: independently is an integer of from 0, 1 or 2;
- r: which may be the same or different, independently represent an integer of from 0 to 10, preferably 1 to 10;
- w: independently represents an integer of from 1 to 10,
for obtaining a polymerizable compound of formula (I), (II) or (III) as defined above.

The reaction may be carried out by mixing, preferably during heating, of components (XII), (XIII) and/or (XIV), and (XVI) in a reaction vessel and reacting the mixture in a single step for providing a compound of the present invention. Preferably, r and t are not 0 at the same time.

The reaction time is not particularly limited and may be selected in the range from 30 minutes to 48 hours. Preferably, the reaction time is selected in the range of from 1 hours to 12 hours, more preferably, from 2 hours to 10 hours.

The reaction temperature is not particularly limited and may be selected in the range from ambient temperature to the boiling temperature of the mixture. Preferably, the reaction temperature is selected in the range of from 50 °C to the 150 °C, more preferably from 70 °C to 130 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dimethyl sulfoxide, toluene, DMF, and ethyleneglycol monomethyl ether.

The reaction may be carried out in the presence of a catalyst. The catalyst may be a phase transfer catalyst. A suitable phase transfer catalyst may be selected from quaternary ammonium and phosphonium salts. Specifically, the phase transfer catalyst may be triethylbenzyl ammonium chloride, benzyltrimethyl ammonium chloride and hexadecyltributylphosphonium bromide. Preferably, triethylbenzyl ammonium chloride may be used.

The amount of the catalyst is not particularly limited and may be selected in a range of from 0.01 to 5 percent by weight, more preferably 0.1 to 3 percent by weight, based on the total weight of components (XI), (XII), and (XIII) present in the reaction mixture.

When component (XVI) is present in the reaction mixture, the reaction may be carried out in the presence of an inhibitor. The inhibitor may be any conventionally known inhibitor which does not interfere with the desired reaction. The inhibitor may be selected from 2,6-di-tert-butyl-p-cresol (BHT), hydroquinone, hydroquinone monomethyl ether, tert.-butyl hydrochinone (TBHQ), TEMPO, and phenothiazin. Preferably, 2,6-di-tert-butyl-p-cresol may be used.

The amount of the inhibitor is not particularly limited and may be selected in a range of from 0.001 to 0.5 percent by weight, more preferably 0.01 to 0.3 percent by weight, based on the total weight of components (XI), (XII), and (XIII) present in the reaction mixture.

The obtained polymerizable compound is may be soluble in organic solvents such as chloroform, DMF and THF and in reactive diluents such as triethyleneglycol dimethacrylate.

According to a third process, a reaction mixture is subjected to an addition reaction for obtaining an addition product. The reaction mixture comprises one or more compounds of formula (XVII), (XVIII) and/or formula (XIX): 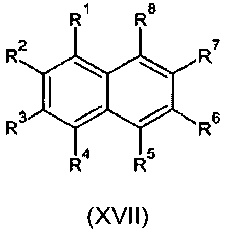 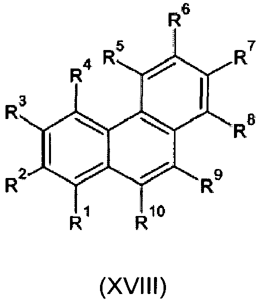 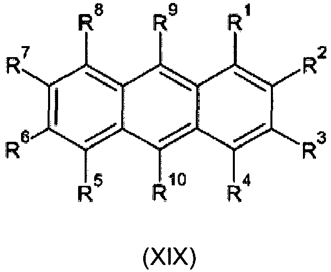 wherein
2 to 4 groups of R¹, R², R³, R⁴, R⁵, R⁶, R⁷, and R⁸, and, if present, R⁹, and R¹⁰ are isocyanatomethyl groups, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

The addition reaction is carried out with a compound of the following formula (XX): 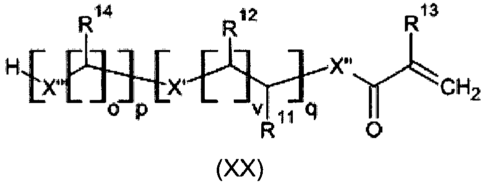 wherein
- X': is -NH-, an oxygen atom or a sulfur atom,
- X": is -NH-, an oxygen atom or a sulfur atom,
- X''': is -NH-, an oxygen atom or a sulfur atom,
- R¹¹ and R¹²: which may be the same or different, independently represent a hydrogen atom or a straight chain or branched C₁₋₆ alkyl group,
- R¹³: represents a hydrogen atom or a straight chain or branched C₁₋₆ alkyl group which may be substituted by a hydroxyl group or carboxyl group,
- R¹⁴: which may be the same or different, independently represent a hydrogen atom, a hydroxyl group, or a straight chain or branched C₁₋₆ alkyl group,
- o: which may be the same or different, independently represent an integer of from 2 to 10,
- p: which may be the same or different, independently is an integer of from 0, 1 or 2;
- q: which may be the same or different, independently represent an integer of from 0 to 10, preferably 1 to 10;
- v: independently represents an integer of from 1 to 10,
for obtaining a polymerizable compound of formula (I), (II) or (III) as defined above.

The reaction may be carried out by mixing, preferably during heating, of components (XVII), (XVIII) and/or (XIX), and (XX) in a reaction vessel and reacting the mixture in a single step for providing a polymerizable composition of the present invention. Preferably, p and q are not 0 at the same time.

The reaction time is not particularly limited and may be selected in the range from 30 minutes to 48 hours. Preferably, the reaction time is selected in the range of from 1 hours to 12 hours, more preferably, from 2 hours to 10 hours.

The reaction temperature is not particularly limited and may be selected in the range from ambient temperature to the boiling temperature of the mixture. Preferably, the reaction temperature is selected in the range of from 50 °C to the 150 °C, more preferably from 70 °C to 130 °C.

The reaction pressure is not particularly limited and may be selected in the range from ambient pressure to an elevated pressure. Preferably, the reaction pressure is ambient pressure.

The reaction may be carried out in the presence or absence of a solvent. Suitable solvent may be selected from aprotic solvents such as dimethyl sulfoxide, toluene, DMF, and ethyleneglycol monomethyl ether. Preferably, the reaction is carried out in the absence of a solvent.

The reaction may be carried out in the presence of a catalyst. The catalyst may be a phase transfer catalyst. A suitable phase transfer catalyst may be selected from quaternary ammonium and phosphonium salts. Specifically, the phase transfer catalyst may be triethylbenzyl ammonium chloride, benzyltrimethyl ammonium chloride and hexadecyltributylphosphonium bromide. Preferably, triethylbenzyl ammonium chloride may be used.

The amount of the catalyst is not particularly limited and may be selected in a range of from 0.01 to 5 percent by weight, more preferably 0.1 to 3 percent by weight, based on the total weight of components (XVII), (XVIII), and (XIX) present in the reaction mixture.

When component (XX) is present in the reaction mixture, the reaction may be carried out in the presence of an inhibitor. The inhibitor may be any conventionally known inhibitor which does not interfere with the desired reaction. The inhibitor may be selected from 2,6-di-tert-butyl-p-cresol (BHT), hydroquinone, hydroquinone monomethyl ether, tert.-butyl hydrochinone (TBHQ), TEMPO, and phenothiazin. Preferably, 2,6-di-tert-butyl-p-cresol may be used.

The amount of the inhibitor is not particularly limited and may be selected in a range of from 0.001 to 0.5 percent by weight, more preferably 0.01 to 0.3 percent by weight, based on the total weight of components (XVII), (XVIII), and (XIX) present in the reaction mixture.

The obtained methacrylate terminated macromer is may be soluble in organic solvents such as chloroform, DMF and THF and in reactive diluents such as triethyleneglycol dimethacrylate.

According to a specific embodiment, a polymerizable compound of formula (I), (II) or (III), which contains hydroxyl groups in any of R¹³, R¹⁴, and/or R¹⁵, may be further reacted with an isocyanate compound, a diisocyanate compound, isothiocyanate compound, a carboxylic acid halogenide compound, a cyclic carboxylic acid anhydride or a carboxylic acid compound for preparing a substituted hydroxyl group. Preferably, a polymerizable compound of formula (I), (II) or (III), which contains hydroxyl groups in any of R¹⁴, and/or R¹⁵ is substituted.

An isocyanate compound may be aliphatic and/or aromatic. As examples of an isocyanate, methylisocyanate (MIC), allyl isocyanate, 2-isocyanatoethyl methacrylate, 2-isocyanatoethyl acrylate, 3-isocyanatopropyl methacrylate, 4-isocyanatobutyl methacrylate, and 6-isocyanatohexyl methacrylate may be mentioned.

A diisocyanate compound may be aliphatic and/or aromatic. As examples of a diisocyanate compound, methylene diphenyl diisocyanate (MDI), toluene diisocyanate (TDI), hexamethylene diisocyanate (HDI) and isophorone diisocyanate (IPDI) may be mentioned.

An isothiocyanate compound may be aliphatic and/or aromatic. As examples of an isothiocyanate, methylisothiocyanate, allyl isothiocyanate, 2-isothiocyanatoethyl methacrylate, 2-isothiocyanatoethyl acrylate, 3-isothiocyanatopropyl methacrylate, 4-isothiocyanatobutyl methacrylate, and 6-isothiocyanatohexyl methacrylate may be mentioned.

A carboxylic acid halogenide compound may preferably be a carboxylic acid chloride or a carboxylic acid bromide. As examples of a carboxylic acid halogenide compound, acryloyl chloride, methacryloyl chloride, acryloyl bromide, methacryloyl bromide may be mentioned.

A cyclic carboxylic acid anhydride may be selected from pentandioic anhydride, phthalic anhydride, itaconic anhydride or maleic anhydride.

A carboxylic acid compound may be alihatic and/or aromatic and may be selected from acrylic acid and methacrylic acid.

The present invention will now be further illustrated based on the following examples.

### Examples

### Example1 - Naphthalene-2,7-diylbis(oxy))bis(ethane-2,1-diyl)bis(2-methacrylate)

### 2,2'-(naphthalene-2,7-diylbis(oxy))diethanol

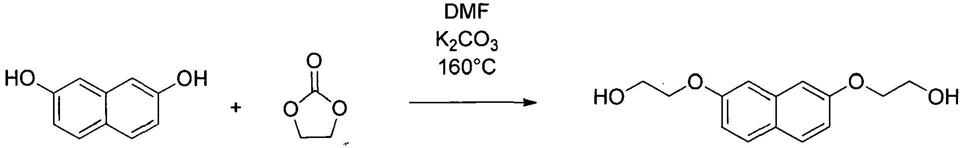

5.00 g 2,7-dihydroxynaphthalene (31.21 mmol), 6.05 g ethylene carbonate (68.66 mmol; 2.2 eq.), and 8.62 g potassium carbonate (68.43 mmol) were solved in 100 ml DMF. The mixture was heated to 160°C for 2 h. After that, the mixture was concentrated via rotary evaporator before the product was precipitated in 1000 mL NaOH (1M). The slightly brown solid was filtrated and washed several times with 500 mL water. The obtained dark brown solid was dried under high vacuum. 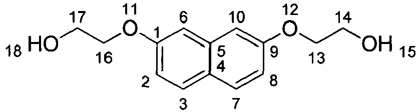 Yield: 5.02g (64%)

| | |
|---|---|
| Elementary analysis: | theor. [%] C: 68.74; H: 6.29 |
| | pract. [%] C: 67.29; H: 6.28 |
| ¹H-NMR [ppm]: | [300MHz; DMSO-d₆]: δ 7.70 (d, 2H, Pos. 3, 7); δ 7.20 (d,2H, Pos. 6, 10); δ 7.00 (dd, 2H, Pos. 2, 8); δ 4.91 (s, 2H, Pos. 15, 18); δ 4.08 (t, 4H, Pos. 13, 16); δ3.78 (m broad,4H, Pos. 14, 17) |
| ¹³C-NMR[ppm]: | [150MHz; DMSO-d₆]: δ 157.11 (C1, 9); δ 135.77 (C5); δ128.97 (C3, 7); δ 123.68 (C4); δ 115.98 (C2), 8;δ 106.10(C6), 10; δ 64.47 (C13, 16); δ 59.57 (C14, 17) |

### (Naphthalene-2,7-diylbis(oxy))bis(ethane-2,1-diyl)bis(2-methacrylate)

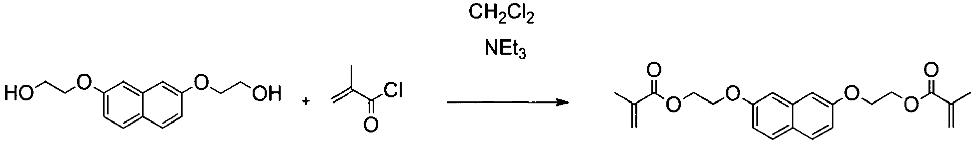

5.00 g 2,2'-(naphthalene-2,7-diylbis(oxy))diethanol (20.14 mmol) and 4.89 g triethylamine (19.33 mmol; 2.4 eq.) were added to 80 mL CH₂Cl₂ and cooled down to 0°C. Under high stirring, 5.05 g methacryloyl chloride (48.33 mmol; 2.4 eq.) solved in 20 mL CH₂Cl₂, was added slowly to the reaction mixture. The mixture was allowed to stay at 0°C for 60 min. before the mixture was stirred for 24 h at room temperature. After that, the mixture was concentrated via rotary evaporator. The mixture was washed two times with 100 mL Na₂CO₃ (1M) and one time with 100 mL water. The organic layer was dried with MgSO₄.

### Example 2-(Naphthalene-2,7-diylbis(oxy))bis(hexane-6,1-diyl)bis(2-methacrylate)

### 2,7-bis((6-bromohexyl)oxy)naphthalene

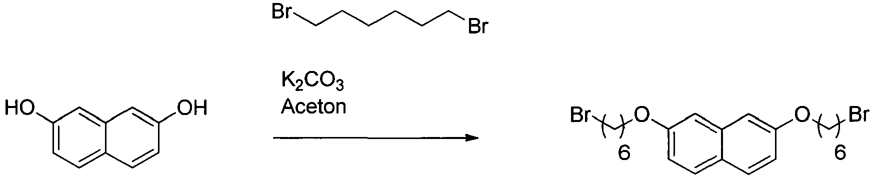

5 g 2,7-dihydroxynaphthalene (31.21 mmol) and 9.49 g potassium carbonate (68.60 mmol) were added in a 500 ml one neck flask and solved with 250 ml Acetone. The reaction mixture was refluxed for 2 h and cooled down to room temperature, before 60.92 g 1,6-dibromohexane (249.70 mmol) was added. After that, the reaction mixture was refluxed for 48h. Next, the solvent was removed via rotary evaporator and the residual was solved in 80ml CHCl₃, before the solution was washed with 2M HCI (2x80 ml) and H₂O (2x80 ml). The collected organic layers were dried over MgSO₄. After removal of the solvent via rotary evaporator a brown/green mixture was obtained. To this, 300 mL Ethanol was added whereat a yellow/green solid precipitated. The product was obtained as a colorless powder via column chromatography (CHCl₃:n-hexane= 9:1). 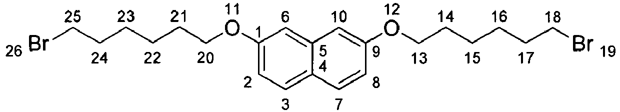 Yield: 4.55g (29%)

| | |
|---|---|
| Elementary analysis: | theor. [%] C: 54.34; H: 6.22 |
| | pract. [%] C: 54.20; H: 6.21 |
| ¹H-NMR [ppm]: | [300MHz; CDCl₃]: δ 7.64 (d, 2H, Pos. 3, 7); δ 7.01 (d, 2H,Pos. 6, 10);δ 6.95 (d, 2H, Pos. 2, 8); δ 4.05 (t, 4H, Pos. 13; 20); δ 3.42 (t, 4H, Pos. 18, 25); δ1.92 - 1.82(m, 8H, Pos. 14, 17, 21, 24); δ 1.52 (m, 4H, Pos. 15, 16,22, 23) |
| ¹³C-NMR [ppm]: | [150MHz; [CDCl₃]: δ 157.78 (C1, 9); δ 136.13 (C5); δ 129.28 (C4); δ 116.43 (C2, 8); δ 106.21 (C6, 10);δ 67.88 (C13, 20); δ 34.04 (C18, 25); δ 32.91 (C17, 24); δ 29.30 (C14, 21); δ 28.16 (C16, 23); δ 25.58 (C15, 22) |
| ESI (MS): | 487:1 M+H⁺ [m/z] |

### (Naphthalene-2,7-diylbis(oxy))bis(hexane-6,1-diyl)bis(2-methacrylate)

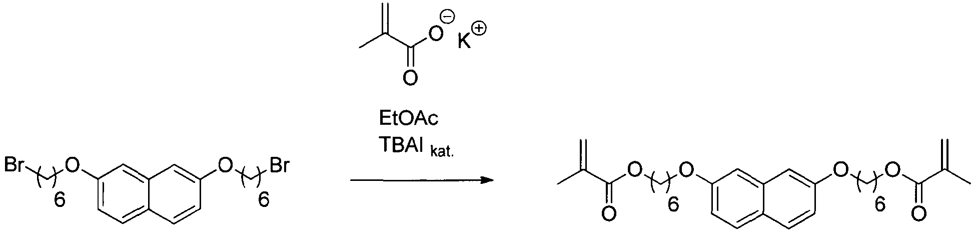

3.6 g 2,7-bis((6-bromohexyl)oxynaphthalene) (7.40 mmol), 3.67 g potassium methacrylate (29.61 mmol; 4 eq.) and 0.054 g tetrabutylammonium iodide (0.1 mmol) were solved in 60 mL ethylacetate. The suspension was heated to 75°C for 24 h. After that, the suspension was filtered and the solvent was removed via rotary evaporator. The residual was solved in 50 mL diethylether and some crystals BHT were added. The solution was washed with 50 mL 1M HCl, 50 mL 1M NaOH and 50 mL water. The organic layer was dried over MgSO₄. The product was obtained as slightly yellow viscous oil. 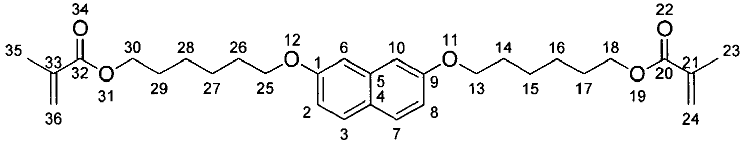 Yield: 2.5g (68%)

| | |
|---|---|
| Elementary analysis: | theor. [%] C: 72.55; H: 8.12 |
| | pract. [%] C: 72.55; H: 8.01 |
| ¹H-NMR [ppm]: | [300MHz; CDCl₃]: δ 7.63 (d, 2H, Pos. 3, 7); δ 7.00 (d, 2H,Pos. 6, 10);δ 6.95 (dd, 2H, Pos. 2, 8); δ 6.08 (m, 2H, Pos. 24a; 36a); δ 5.52 (m, 2H, Pos. 24b, 36b); δ4.15 (t, 4H, Pos. 13, 25); δ 4.04 (t, 4H, Pos. 18, 30);δ 1.93 (m, 6H, Pos. 23, 35); δ 1.84 (m, 4H, Pos. 14, 26);δ 1.71 (m, 4H, Pos. 17, 29); δ 1.50 (m, 8H, Pos. 15, 16,27, 28) |
| ¹³C-NMR [ppm]: | [150MHz; CDCl₃]: δ 167.72 (C20, 32); δ 157.78 (C1, 9); δ136.99 (C21, 33); δ 136.13 (C5); δ 129.25 (C3, 7);δ 125.41 (C24, 36); δ 124.37 (C4); δ 116.41 (C2, 8); δ 106.19 (C6, 10); δ 67.92 (C13, 25); δ 64.86 (C18, 30); δ 29.36 (C14,26); δ 28.78 (C15, 16, 27, 28); δ 18.53 (C23, 35) |
| ESI (MS): | 497.5 M+H⁺ [m/z] |

### Example 3 - 4-((7-((4-(methacryloyloxy)butane-2-yl)oxy)naphthalen-2-yl)oxy)butan-2-yl methacrylate

### 2-((4-bromobutane-2-yl)oxy)7-(3-bromobutoxy)naphthalene

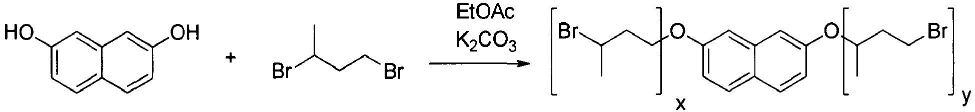

5 g 2,7-dihydroxynaphthalene (31.21 mmol) and 9.49 g potassium carbonate (68.60 mmol) were added in a 500 ml one neck flask and solved with 250 ml Acetone. The reaction mixture was refluxed for 2 h and cooled down to room temperature, before 40.43 g 1,3-dibromobutane (187.26 mmol) was added. After that, the reaction mixture was refluxed for 24 h. Next, the solvent was removed via rotary evaporator The product was obtained as slightly yellow solid via column chromatography (CHCl₃:n-hexane= 10:1; R_{f} = 0.8).
Yield: 7.50g (55%)

| | |
|---|---|
| Elementary analysis: | theor. [%] C: 50.26; H: 5.15 |
| | pract. [%] C: 56.69; H: 5.58 |
| EI (MS): | 430 [M]+ [m/z] |

### 4-((7-((4-(methacryloyloxy)butane-2-yl)oxy)naphthalen-2-yl)oxy)butane-2-yl-methacrylate

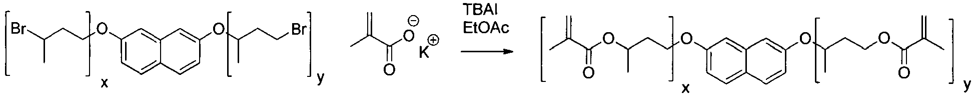

7.5 g 2-((4-bromobutane-2-yl)oxy)7-(3-bromobutoxy)naphthalene (17.43 mmol), 8.66 g potassium methacrylate (69.73 mmol; 4eq.) and 0.110 g tetrabutylammonium iodide (0.35 mmol) were solved in 200 mL ethylacetate. The suspension was heated to 75°C for 24 h. After that, the suspension was filtered and the solvent was removed via rotary evaporator. The residual was solved in 100 mL diethylether and some crystals BHT were added. The solution was washed with 100 mL 1M HCl, 100mL 1M NaOH and 100 mL water. The organic layer was dried over MgSO₄. The product was obtained as slightly yellow viscous oil.
Yield: 4.60g (59%)

| | |
|---|---|
| ESI (MS): | 441 [M+H]+; 458.3 [M+NH4+] [m/z] |

### Example 4 - (Naphthalene-2,7-diylbis(oxy))bis(propane-3,1-diyl)bis(2-methacrylate)

### 3,3'-(naphthalene-2,7-diylbis(oxy))bis(propane-1-ol)

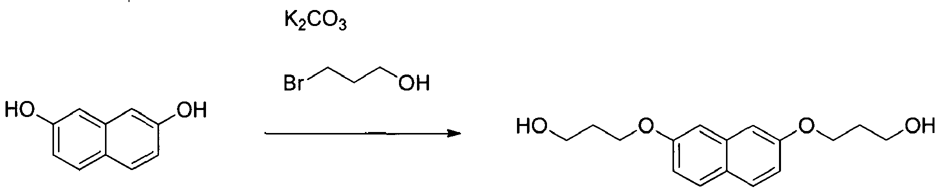

2.5 g 2,7-dihydroxynaphthalene (15.60 mmol) and 4.89 g potassium carbonate (35.43 mmol) were solved in 125 ml acetone. The reaction mixture was refluxed for 2 h and cooled down to room temperature, before 4.77 g 1,3-dibrompropane (34.44 mmol) was added. After that, the reaction mixture was refluxed for 24 h. Next, the solvent was removed via rotary evaporator. The product was obtained as colorless solid via column chromatography (ethyl acetate:n-hexane = 10:1). 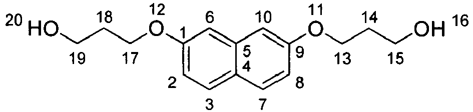 Yield: 0.9g (21%)

| | |
|---|---|
| ¹H-NMR [ppm]: | [300MHz; Methanol-d₄ ]: δ 7.63 (d, 2H, Pos. 3, 7); δ 7.13(d, 2H, Pos. 6, 10); δ 6.96 (dd, 2H, Pos. 2, 8); δ 4.16(t, 4H, Pos. 13, 17); δ 3.78 (t, 4H, Pos. 15, 19);δ 2.03 (quint., 4H, Pos. 14, 18) |
| ¹³C-NMR [ppm]: | [75MHz; Methanol-d₄]: δ 159.07 (C1,9); δ 137.72 (C23,27);δ 130.10 (C3,7); δ 125.89 (C4); δ 117.30 (C2,8); δ 107.31 (C6,10); δ 65.81 (C13, 17) δ 59.81 (C15,19); δ 33.53 (C14, 18) |
| EI (MS): | 276 M; 277 M+H⁺ |

### (Naphthalene-2,7-diylbis(oxy))bis(propane-3,1-diyl)bis(2-methacrylate)

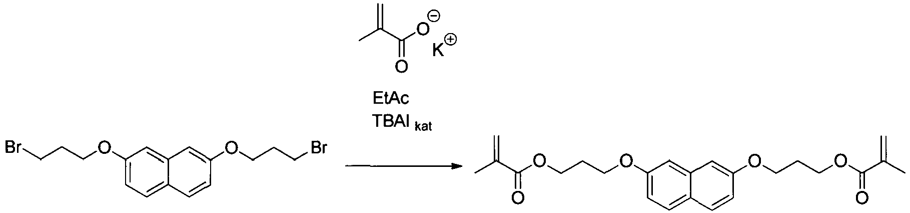

4.00 g 2,7-bis(3-bromopropoxy)naphthalene) (9.94 mmol), 4.93 g potassium methacrylate (39.76 mmol; 4 eq.) and 0.073 g tetrabutylammonium iodide (0.2 mmol) were solved in 60 mL ethyl acetate. The suspension was heated to 75°C for 24 h. After that, the suspension was filtered and the solvent was removed via rotary evaporator. The residual was solved in 50 mL diethyl ether and some crystals BHT were added. The solution was washed with 50 mL 1M HCI, 50 mL 1M NaOH and 50 mL water. The organic layer was dried over MgSO₄. The product was obtained as colorless solid. 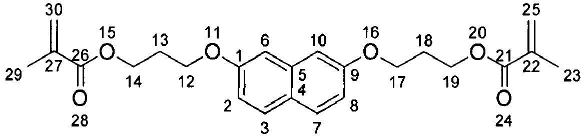 Yield: 2.73g (66%)
Mp: ∼ 60°C (opt.)

| | |
|---|---|
| Elementary analysis: | theor. [%] C: 69.88; H: 6.84 |
| | pract. [%] C: 69.88; H: 6.68 |
| ¹H-NMR [ppm]: | [300MHz; CDCl₃]: δ 7.58 (d, 2H, Pos. 3, 7); δ 6.96 (d, 2H,Pos. 6, 10);δ 6.93 (dd, 2H, Pos. 2, 8); δ 6.05 (m, 2H, Pos. 25a; 30a); δ 5.49 (m, 2H, Pos. 25b, 30b); δ4.31 (t, 4H, Pos. 12, 17); δ 4.09 (t, 4H, Pos. 14, 19);δ 2.15 (qui., 4H, Pos. 13, 18); δ 1.88 (m, 6H, Pos. 23, 29) |
| ¹³C-NMR [ppm]: | [150MHz; CDCl₃]: δ 167.57 (C21, 26); δ 157.53 (C1, 9); δ136.50 (C22, 27); δ 136.00 (C5); δ 129.37 (C3, 7);δ 125.73 (C25, 30); δ 124.81 (C4); δ 116.47 (C2, 8); δ 106.23 (C6, 10); δ 64.58 (C12, 17); δ 61.78 (C14, 19); δ 28.88 (C13, 18); δ 18.53 (C23, 29) |
| ESI (MS): | 413.2 M+H⁺ ; 435.2 M+Na⁺ [m/z] |

### Example 5 - {[(((naphthalene-2,7-diylbis(oxy))bis(propane-3,1-diyl))bis(oxy)) bis(carbonyal)] bis(azanediyl)}bis(ethane-2,1-diyl)bis(2-methacrylate)

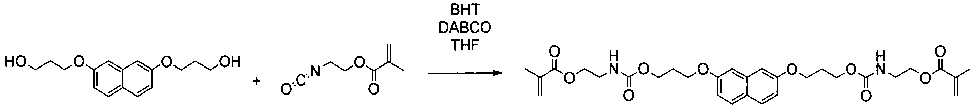

2 g 3,3-(naphthalene-2,7-diylbis(oxy))bis(propane-1-ol) (7.23 mmol), 2.69 g isocyanato ethylmethacrylate (17.37 mmol; 2.4 eq.), 0.081 g 1,4-diazabicyclo[2.2.2]octane (0.723 mmol) and 0.0031 g 2,6-di-tert-butyl-p-kresol (0.014 mmol) were solved in 50 mL THF. The solution was heated to 75°C for 24 h. After that, the solvent was removed via rotary evaporator. The product was obtained as colorless solid via column chromatography (ethyl acetate: n-hexane = 2:1). 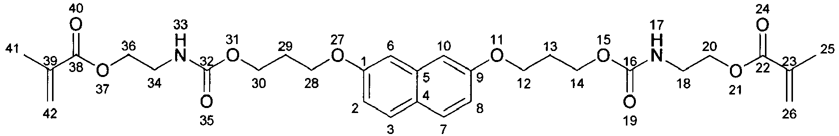 Yield: 2.12g (52%)

| | |
|---|---|
| Elementary analysis: | theor. [%] C: 61.42; H: 6.53; N: 4.78 |
| | pract. [%] C: 61.46; H: 6.44; N: 4.77 |
| ¹H-NMR [ppm]: | [300MHz; CDCl₃]: δ 7.64 (d, 2H, Pos. 3, 7); δ 7.00 (d, 2H, Pos. 6, 10);δ 6.95 (dd, 2H, Pos. 2, 8); δ 6.09 (m, 2H, Pos. 26a; 42a); δ 5.56 (m, 2H, Pos. 26b, 42b); δ4.93 (s, 2H, Pos. 17, 33); δ 4.29 (t, 4H, Pos. 20, 36);δ 4.21 (t, 4H, Pos. 12, 28); δ 4.12 (t, 4H, Pos. 14, 30);δ 3.49 (q, 4H, Pos. 18, 34); δ 2.15 (quin., 4H, Pos. 13, 29); δ 1.91 (m, 6H, Pos. 25, 41) |
| ¹³C-NMR [ppm]: | [150MHz; CDCl₃]: δ 167.50 (C22, 38); δ 157.57 (C16, 32); δ156.63 (C1, 9); δ 136.15 (C5); δ 136.03 (C23, 39);δ 129.37 (C3, 7); δ 126.28 (C26, 42); δ 124.58 (C4); δ 116.47 (C2, 8); δ 106.29 (C6, 10); δ 64.55 (C20, 36); δ 63.93 (C12,28); δ 62.17 (C14, 30); δ 40.43 (C18, 34); δ 29.23 (C13, 29); δ 18.52 (C25, 41) |
| ESI (MS): | 587.5 M+H+ [m/z] |

### Example 6 - (Naphthalene-1,5-diylbis(oxy))bis(ethane-2,1-diyl)bis(2-methacrylate)

### 2,2'-(naphthalene-1,5-diylbis(oxy))diethanol

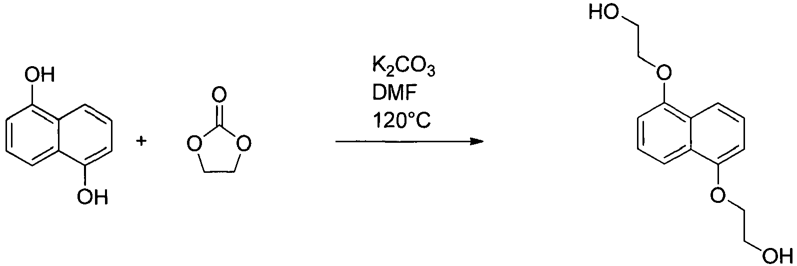

5.00 g 1,5-dihydroxynaphthalene (31.21 mmol), 6.05 g ethylene carbonate (68.66 mmol; 2.2 eq.), and 8.62 g potassium carbonate (68.43 mmol) were solved in 100 ml DMF. The mixture was heated to 160°C for 2 h. After that, the mixture was concentrated via rotary evaporator before the product was precipitated in 1000 mL NaOH (1M). The slightly brown solid was filtrated and washed several times with 500 mL water. The obtained slightly brown solid was dried under high vacuum. 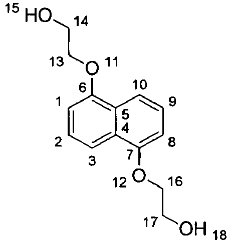 Yield: 4.65g (60%)

| | |
|---|---|
| Elementary analysis: | theor. [%] C: 67.73; H: 6.50 |
| | pract. [%] C: 66.85; H: 6.24 |
| ¹H-NMR [ppm]: | [300MHz; DMSO-d₆]: δ 7.80 (d, 2H, Pos. 3, 10); δ 7.37 (t, 2H, Pos. 2, 9); δ 6.98 (d, 2H, Pos. 1, 8); δ 4.98 (s, 2H, Pos. 15, 18); δ 4.14 (t, 4H, Pos. 13, 16); δ 3.86 (t, 4H, Pos. 14, 17) |
| ¹³C-NMR [ppm]: | [75MHz; DMSO-d₆]: δ 154.07 (C7,6); δ 126.07 (C4,5); δ 125.29 (C2,9); δ 113.96 (C3,10); δ 105.78 (C1,8); δ 69.92 (C13, 16); δ 59.74 (C14, 17) |
| EI (MS): | 248 M; 249 M+H⁺ |

### (Naphthalene-1,5-diylbis(oxy))bis(ethane-2,1-diyl)bis(2-methacrylate)

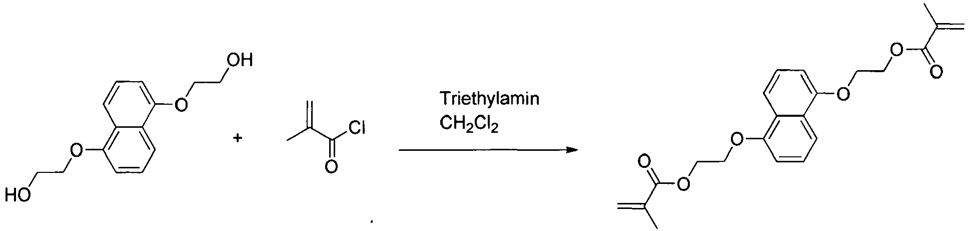

2.00 g 2,2'-(naphthalene-1,5-diylbis(oxy))diethanol (8.05mmol) and 1.95g triethylamine (19.33 mmol; 2.4 eq.) were added to 50 mL CH₂Cl₂ and cooled down to 0°C. Under high stirring, 2.02 g methacroyl chloride (19.33 mmol; 2.4 eq.) solved in 10 mL CH₂Cl₂, were added slowly to the reaction mixture. The mixture was allowed to stay at 0°C for 60 min. before the mixture was stirred for 24 h at room temperature. After that, the mixture was concentrated via rotary evaporator. The mixture was washed two times with 100 mL Na₂CO₃ (1M) and one time with 100 mL water. The organic layer was dried with MgSO₄. The product was obtained as colorless solid via column chromatography (CH₂Cl₂:n-hexane = 4:1). 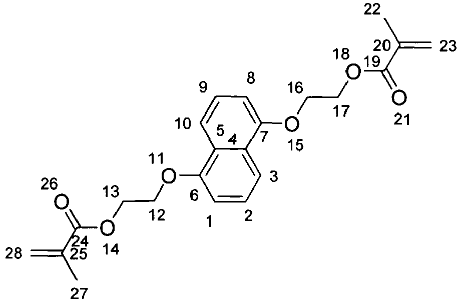 Yield: 0.87g (28%)

| | |
|---|---|
| ¹H-NMR [ppm]: | [300MHz; CDCl₃]: δ 7.84 (d, 2H, Pos. 3, 10); δ 7.35(t, 2H, Pos. 2, 9); δ 6.85 (d, 2H, Pos. 1, 8); δ 6.14(m, 2H, Pos. 23a/28a); δ 5.56 (m, 2H, Pos. 23b, 23b);δ 4.62 (m, 4H, Pos. 13, 17); δ 4.36 (m, 4H, Pos. 12,16);δ 1.94 (dd, 6H, Pos. 22, 27) |
| ¹³C-NMR [ppm]: | [75MHz; CDCl₃]: δ 167.59 (C19,24); δ 154.33 (C6,7);δ 136.24 (C20,25); δ 127.01 (C4,5); δ 126.26 (C2,9); δ 125.38 (C23,28); δ 115.06 (C3,10); δ 106.11 (C1,8); δ 66.61 (C12,16);δ 63.22 (C13,17); δ 18.53 (C22,27) |

### Application Example 1

(Naphthalene-2,7-diylbis(oxy))bis(hexane-6,1-diyl)bis(2-methacrylate) (FSE4C6KM) according to example 2 was mixed with 1.0 mol-% camphorquinone (CQ) and 1.3 mol-% ethyl 4-dimethylaminobenzoate (DMABE) until a homogenous mixture was obtained. 6 samples were taken and prepared to cured specimen according to ISO 4049. Subsequently, flexural strengths and E-modulus of the samples, measuring (25 ± 2) mm x (2 ± 0.2) mm x (2 ± 0.2) mm were tested according to ISO 4049.

The procedure was repeated with a mixture of FSE4C6KM and 30 wt.-% triethylene glycol dimethacrylate (FSE4C6KM-T30).

| **Batch** | | FSE4C6KM | FSE4C6KM-T30 |
|---|---|---|---|
| Concentration TEGDMA | wt.-% | 0 | 30 |
| Viscosity | Pa·s | 0.51 | - |
| Refractive Index | - | 1.539 | - |
| Flexural Strength | MPa | 77.5 ± 5.3 | 83.4 ± 8.6 |
| E-modulus | MPa | 1369 ± 53 | 1636 ± 68 |

### Comparative Example 1

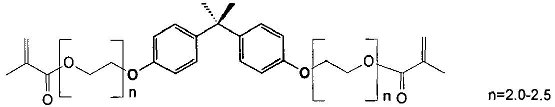

2,2-Bis-[4-(methacryloyloxydiethoxy)phenyl]-propane (EBA) was mixed with 1.3 mol-% campherquinone (CQ) and 1.3 mol-% ethyl 4-dimethylaminobenzoate (DMABE) until a homogenous mixture was obtained. 6 samples were taken and prepared to cured specimen according to ISO 4049. Subsequently, flexural strengths and E-modulus of the samples, measuring (25 ± 2) mm x (2 ± 0.2) mm x (2 ± 0.2) mm were tested according to ISO 4049. The procedure was repeated with a mixture of EBA and 30 wt.-% triethylene glycol dimethacrylate (AG 18-92-1).

| **Batch** | | EBA | AG 18-92-1 |
|---|---|---|---|
| Concentration TEGDMA | wt.-% | 0 | 30 |
| Viscosity | Pa·s | 0.65 | 0.158 |
| Refractive Index | - | 1.535 | 1.513 |
| Flexural Strength | MPa | 76.6 ± 4.6 | 95.3 ± 0.7 |
| E-modulus | MPa | 1223 ± 116 | 1923 ± 48 |

## Claims

1. Dental composition comprising a polymerizable compound of the following formula (I), (II) and/or formula (III): 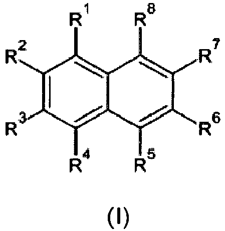 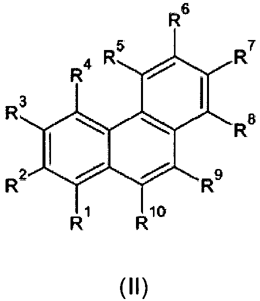 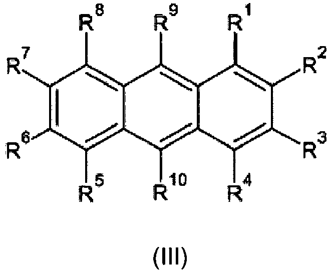 wherein
2 to 4 groups of R¹, R², R³, R⁴, R⁵, R⁶, R⁷, and R⁸, and, if present, R⁹, and R¹⁰, which are independent from each other, represent a group of the following formula (IV), (V), or (VI): 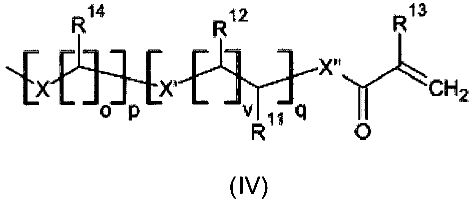 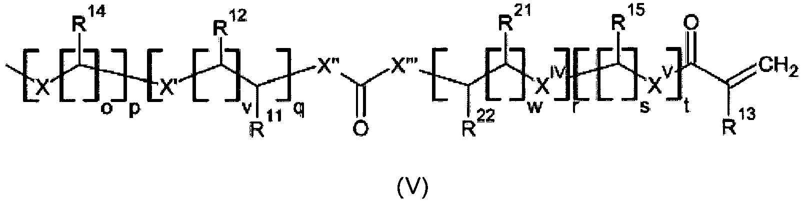 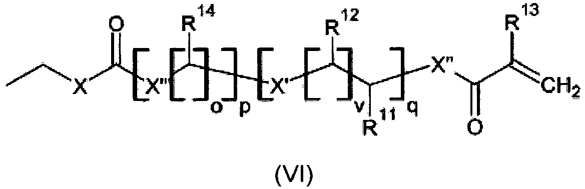 wherein
X is -NH-, an oxygen atom or a sulfur atom,
X' is -NH-, an oxygen atom or a sulfur atom,
X" is -NH-, an oxygen atom or a sulfur atom,
X"' is -NH-, an oxygen atom or a sulfur atom,
X^{IV} is -NH-, an oxygen atom or a sulfur atom,
X^{V} is -NH-, an oxygen atom or a sulfur atom,
R¹¹, R¹², R²¹ and R²² which may be the same or different, independently represent a hydrogen atom or a straight chain or branched C₁₋₆ alkyl group,
R¹³ represents a hydrogen atom or a straight chain or branched C₁₋₆ alkyl group which may be substituted by a hydroxyl group or carboxyl group,
R¹⁴ and R¹⁵ which may be the same or different, independently represent a hydrogen atom, a hydroxyl group which may be substituted, or a straight chain or branched C₁₋₆ alkyl group,
o and s which may be the same or different, independently represent an integer of from 2 to 10,
p and t which may be the same or different, independently is an integer of from 0, 1 or 2;
q and r which may be the same or different, independently represent an integer of from 0 to 10;
v and w which may be the same or different, independently represent an integer of from 1 to 10
and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups.

2. The dental composition according to claim 1, which comprises a compound of formula (I).

3. The dental composition according to claim 1, wherein 2 groups of R¹, R², R³, R⁴, R⁵, R⁶, R⁷, and R⁸, and, if present, R⁹, and R¹⁰, represent a group of the formula (IV).

4. The dental composition according to claim 3, wherein the remaining groups of R¹, R², R³, R⁴, R⁵, R⁶, R⁷, and R⁸, and, if present, R⁹, and R¹⁰ represent a hydrogen atom.

5. The dental composition according to anyone of the preceding claims, wherein R² and R⁷, which may be the same or different, represent a group of formula (IV).

6. The dental composition according to claim 4, wherein R¹, R³, R⁴, R⁵, R⁶ and R⁸ represent a hydrogen atom.

7. The dental composition according to claim 1, which comprises a compound of formula (II).

8. The dental composition according to any one of the preceding claims which further comprises a filler and/or a solvent, and which preferably further comprises a polymerization initiator system.

9. The dental composition according to any one of the preceding claims, which is selected from a dental adhesive composition, a dental pit and fissure sealer, a dental composite, and a root canal filling composition.

10. Process for the preparation of a dental composition according to claim 1, comprising the steps of
(a) subjecting a reaction mixture comprising
(a1) one or more compounds of formula (VII), (VIII) and/or formula (IX): 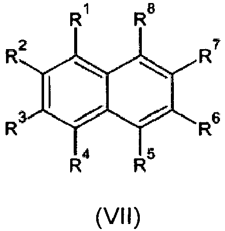 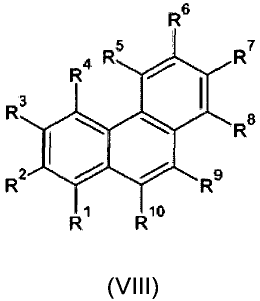 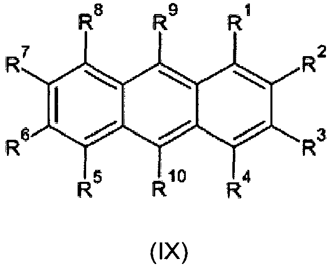 wherein
2 to 4 groups of R¹, R², R³, R⁴, R⁵, R⁶, R⁷, and R⁸, and, if present, R⁹, and R¹⁰ are -XH groups wherein X is -NH-, an oxygen atom or a sulfur atom, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, and
(a2) epichlorohydrine, ethylene carbonate or a compound of the formula ZRZ', wherein Z and Z' independently are leaving groups and R is a straight chain or branched alkylene group;
to an addition reaction for obtaining an addition product; and
(b1) reacting the addition product of epichlorohydrine with a compound of the following formula (XI): 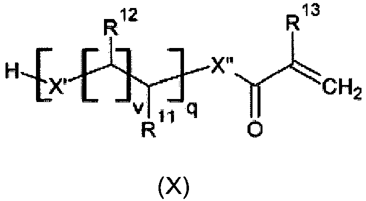 wherein
R¹¹ and R¹² which may be the same or different, independently represent a hydrogen atom or a straight chain or branched C₁₋₆ alkyl group,
R¹³ represents a hydrogen atom or a straight chain or branched C₁₋₆ alkyl group which may be substituted by a hydroxyl group or carboxyl group,
X' is -NH-, an oxygen atom or a sulfur atom,
X" is -NH-, an oxygen atom or a sulfur atom, and
q independently represents an integer of from 0 to 10,
v independently represents an integer of from 1 to 10,
for obtaining a polymerizable compound of formula (I), (II) or (III) as defined in claim 1;
(b2) reacting the addition product of ethylene carbonate with a compound of the following formula (XI) 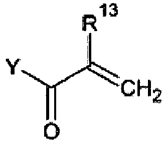 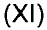 wherein Y is a leaving group and R¹³ represents a hydrogen atom or a straight chain or branched C₁₋₆ alkyl group, or wherein Y and R¹³ form together with the carbon atoms to which they are bound a cyclic lactone ring or carboxylic acid anhydride ring having 4 to 8 carbon atoms;
for obtaining a polymerizable compound of formula (I), (II) or (III) as defined in claim 1; or
(b3) reacting the addition product of a compound of the formula ZRZ', wherein Z and Z' independently are leaving groups and R is a straight chain or branched alkylene group, with a compound of formula (XI) wherein Y is OH.

11. Process for the preparation of a dental composition according to claim 1, comprising subjecting a reaction mixture comprising one or more compounds of formula (XII), (XIII) and/or formula (XIV): 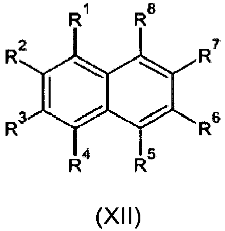 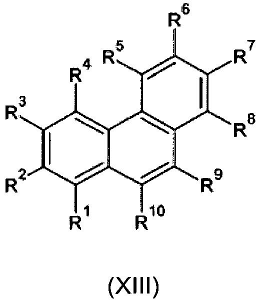 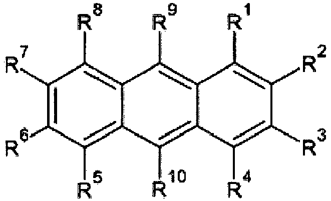 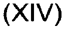 wherein
2 to 4 groups of R¹, R², R³, R⁴, R⁵, R⁶, R⁷, and R⁸, and, if present, R⁹, and R¹⁰ are independently a group of the following formula (XV): 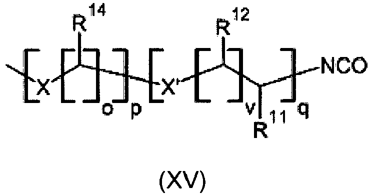 wherein
X is -NH-, an oxygen atom or a sulfur atom,
X' is -NH-, an oxygen atom or a sulfur atom,
R¹¹ and R¹² which may be the same or different, independently represent a hydrogen atom or a straight chain or branched C₁₋₆ alkyl group,
R¹⁴ which may be the same or different, independently represent a hydrogen atom, a hydroxyl group, or a straight chain or branched C₁₋₆ alkyl group,
o independently represent an integer of from 2 to 10,
p independently is an integer of from 0, 1 or 2;
q independently represent an integer of from 0 to 10;
v independently represents an integer of from 1 to 10,
and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, to an addition reaction with a compound of the following formula (XVI): 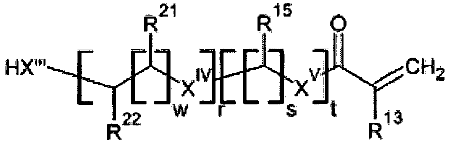 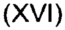 wherein
X"' is -NH-, an oxygen atom or a sulfur atom,
X^{IV} is -NH-, an oxygen atom or a sulfur atom,
X^{V} is -NH-, an oxygen atom or a sulfur atom,
R²¹ and R²² which may be the same or different, independently represent a hydrogen atom or a straight chain or branched C₁₋₆ alkyl group,
R¹³ represents a hydrogen atom or a straight chain or branched C₁₋₆ alkyl group which may be substituted by a hydroxyl group or carboxyl group,
R¹⁵ which may be the same or different, independently represent a hydrogen atom, a hydroxyl group, or a straight chain or branched C₁₋₆ alkyl group,
s independently represent an integer of from 2 to 10,
t independently is an integer of from 0, 1 or 2;
r which may be the same or different, independently represent an integer of from 0 to 10;
w independently represents an integer of from 1 to 10, for obtaining a polymerizable compound of formula (I), (II) or (III) as defined in claim 1.

12. Process for the preparation of a dental composition according to claim 1, comprising subjecting a reaction mixture comprising one or more compounds of formula (XVII), (XVIII) and/or formula (XIX): 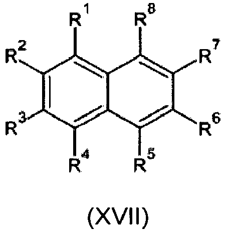 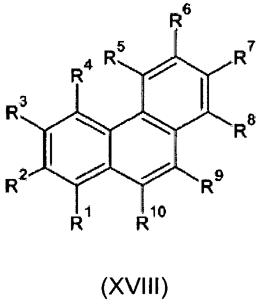 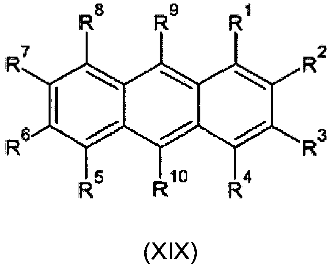 wherein
2 to 4 groups of R¹, R², R³, R⁴, R⁵, R⁶, R⁷, and R⁸, and, if present, R⁹, and R¹⁰ are isocyanatomethyl groups, and the remaining groups of R¹, R², R³, R⁴, R⁵, R⁶, R⁷, and R⁸, and, if present, R⁹, and R¹⁰ independently represent a hydrogen atom, a halogen atom, a straight chain or branched C₁₋₆ alkyl group, a C₃₋₆ cycloalkyl group, or a C₁₋₆ alkyl C₃₋₆ cycloalkyl group, which groups may contain 1 to 3 heteroatoms selected from nitrogen, oxygen, and sulfur atoms or be substituted by 1 to 3 substituents selected from halogen atoms or hydroxy groups, to an addition reaction with a compound of the following formula (XX): 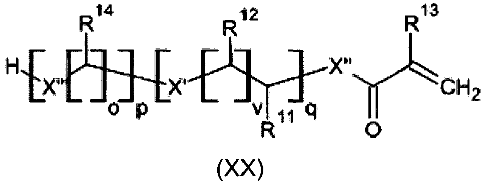 wherein
X' is -NH-, an oxygen atom or a sulfur atom,
X" is -NH-, an oxygen atom or a sulfur atom,
X"' is -NH-, an oxygen atom or a sulfur atom,
R¹¹ and R¹² which may be the same or different, independently represent a hydrogen atom or a straight chain or branched C₁₋₆ alkyl group,
R¹³ represents a hydrogen atom or a straight chain or branched C₁₋₆ alkyl group which may be substituted by a hydroxyl group or carboxyl group,
R¹⁴ which may be the same or different, independently represent a hydrogen atom, a hydroxyl group, or a straight chain or branched C₁₋₆ alkyl group,
o which may be the same or different, independently represent an integer of from 2 to 10,
p which may be the same or different, independently is an integer of from 0, 1 or 2;
q which may be the same or different, independently represent an integer of from 0 to 10;
v independently represents an integer of from 1 to 10,
for obtaining a polymerizable compound of formula (I), (II) or (III) as defined in claim 1.

13. The process according to any one of claims 10 to 12, which further comprises a step of reacting a polymerizable compound of formula (I), (II) or (III) as defined in claim 1, which contains hydroxyl groups in any of R¹³, R¹⁴, and/or R¹⁵, with an isocyanate compound, a diisocyanate compound, isothiocyanate compound, a carboxylic acid halogenide compound, a cyclic carboxylic acid anhydride or a carboxylic acid compound for preparing a substituted hydroxyl group.

14. Dental composition obtainable by the process according to any one of claims 10 to 13.

15. Use of a dental composition according to claim 14 for the preparation of a dental composition as defined in any one of claims 8 to 10.

## Patentansprüche

1. Dentalzusammensetzung, die eine polymerisierbare Verbindung der folgenden Formel (I), (II) und/oder der Formel (III) umfasst: 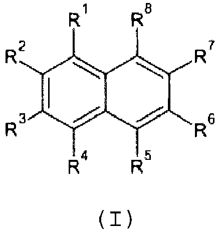 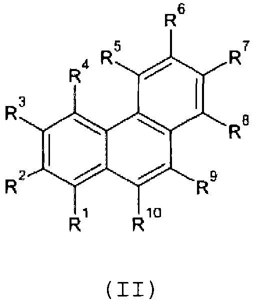 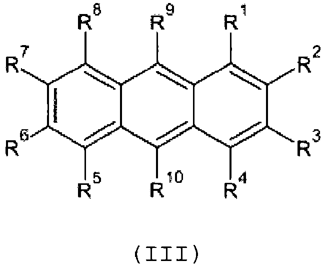 wobei
2 bis 4 Gruppen von R¹, R², R³, R⁴, R⁵, R⁶, R⁷ und R⁸ und, falls vorhanden, R⁹ und R¹⁰, die voneinander unabhängig sind, eine Gruppe der folgenden Formel (IV), (V) oder (VI) darstellen: 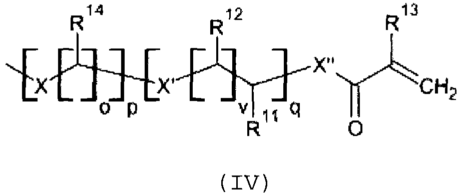 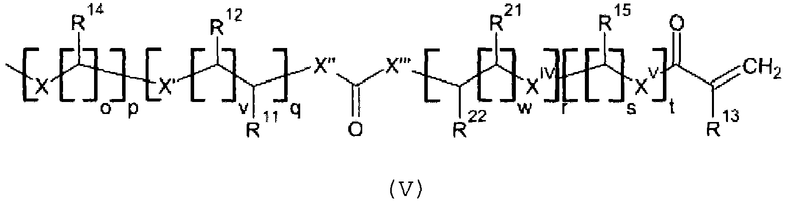 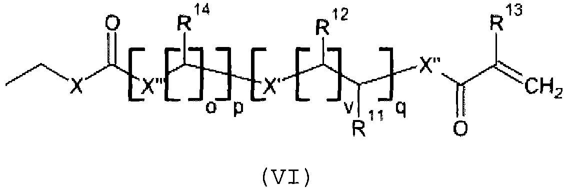 wobei
X -NH-, ein Sauerstoffatom oder ein Schwefelatom ist,
X' -NH-, ein Sauerstoffatom oder ein Schwefelatom ist,
X" -NH-, ein Sauerstoffatom oder ein Schwefelatom ist,
X'" -NH-, ein Sauerstoffatom oder ein Schwefelatom ist,
X^{IV} -NH-, ein Sauerstoffatom oder ein Schwefelatom ist,
X^{V} -NH-, ein Sauerstoffatom oder ein Schwefelatom ist,
R¹¹, R¹², R²¹ und R²²
die gleich oder unterschiedlich sein können, unabhängig ein Wasserstoffatom oder eine gerade Kette oder eine verzweigte C₁₋₆-Alkylgruppe darstellen,
R¹³ ein Wasserstoffatom oder eine gerade Kette oder eine verzweigte C₁₋₆-Alkylgruppe darstellt, die durch eine Hydroxylgruppe oder Carboxylgruppe substituiert sein kann,
R¹⁴ und R¹⁵
die gleich oder unterschiedlich sein können, unabhängig ein Wasserstoffatom, eine Hydroxylgruppe, die substituiert sein kann, oder eine gerade Kette oder eine verzweigte C₁₋₆-Alkylgruppe darstellen können,
o und s die gleich oder unterschiedlich sein können, unabhängig eine ganze Zahl von 2 bis 10 darstellen,
p und t die gleich oder unterschiedlich sein können, unabhängig eine ganze Zahl von 0, 1 oder 2 sind;
q und r die gleich oder unterschiedlich sein können, unabhängig eine ganze Zahl von 0 bis 10 darstellen;
v und w, die gleich oder unterschiedlich sein können, unabhängig eine ganze Zahl von 1 bis 10 darstellen
und die verbleibenden Gruppen von R¹, R², R³, R⁴, R⁵, R⁶, R⁷ und R⁸ und, falls vorhanden, R⁹ und R¹⁰ unabhängig ein Wasserstoffatom, ein Halogenatom, eine gerade Kette oder eine verzweigte C₁₋₆-Alkylgruppe, eine C₃-₆-Cycloalkylgruppe oder eine C₁₋₆-Alkyl-C₃₋₆-Cycloalkylgruppe darstellen, wobei die Gruppen 1 bis 3 Heteroatome enthalten können, die aus Stickstoff-, Sauerstoff- und Schwefelatomen ausgewählt oder durch 1 bis 3 Substituenten substituiert sind, die ausgewählt sind aus Halogenatomen oder Hydroxygruppen.

2. Dentalzusammensetzung nach Anspruch 1, die eine Verbindung der Formel (I) umfasst.

3. Dentalzusammensetzung nach Anspruch 1, wobei 2 Gruppen von R¹, R², R³, R⁴, R⁵, R⁶, R⁷ und R⁸ und, falls vorhanden, R⁹ und R¹⁰ eine Gruppe der Formel (IV) darstellen.

4. Dentalzusammensetzung nach Anspruch 3, wobei die verbleibenden Gruppen von R¹, R², R³, R⁴, R⁵, R⁶, R⁷ und R⁸ und, falls vorhanden, R⁹ und R¹⁰ ein Wasserstoffatom darstellen.

5. Dentalzusammensetzung nach einem der vorstehenden Ansprüche, wobei R² und R⁷, die gleich oder unterschiedlich sein können, eine Gruppe der Formel (IV) darstellen.

6. Dentalzusammensetzung nach Anspruch 4, wobei R¹, R³, R⁴, R⁵, R⁶ und R⁸ ein Wasserstoffatom darstellen.

7. Dentalzusammensetzung nach Anspruch 1, die eine Verbindung der Formel (II) umfasst.

8. Dentalzusammensetzung nach einem der vorstehenden Ansprüche, die ferner einen Füllstoff und/oder ein Lösungsmittel umfasst und die bevorzugt ferner ein Polymerisationsinitiatorsystem umfasst.

9. Dentalzusammensetzung nach einem der vorstehenden Ansprüche, die ausgewählt ist aus einer Dentalhaftzusammensetzung, einem Dentalvertiefungs- und Fissurenversiegler, einem Dentalverbundstoff und einer Wurzelkanalfüllungszusammensetzung.

10. Verfahren zur Herstellung einer Dentalzusammensetzung nach Anspruch 1, das die Schritte aufweist
(a) Unterwerfen eines Reaktionsgemischs, das
(a1) eine oder mehrere Verbindungen der Formel (VII), (VIII) und/oder Formel (IX) umfasst: 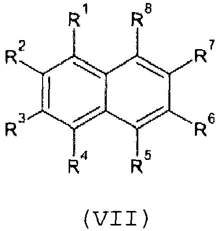 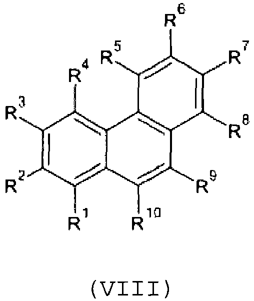 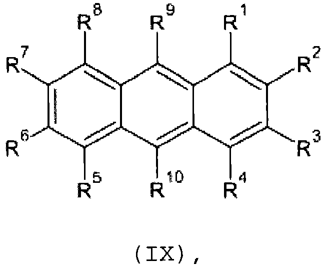 wobei
2 bis 4 Gruppen von R¹, R², R³, R⁴, R⁵, R⁶, R⁷ und R⁸ und, falls vorhanden, R⁹ und R¹⁰ -XH-Gruppen sind, wobei X -NH-, ein Sauerstoffatom oder ein Schwefelatom ist und die verbleibenden Gruppen von R¹, R², R³, R⁴, R⁵, R⁶, R⁷ und R⁸ und, falls vorhanden, R⁹ und R¹⁰ unabhängig ein Wasserstoffatom, ein Halogenatom, eine gerade Kette oder eine verzweigte C₁₋₆-Alkylgruppe, eine C₃₋₆-Cycloalkylgruppe oder eine C₁₋₆-Alkyl-C₃₋₆-Cycloalkylgruppe darstellen, wobei die Gruppen 1 bis 3 Heteroatome enthalten können, die aus Stickstoff-, Sauerstoff- und Schwefelatomen ausgewählt oder durch 1 bis 3 Substituenten substituiert sind, die ausgewählt sind aus Halogenatomen oder Hydroxygruppen, und
(a2) Epichlorhydrin, Ethylencarbonat oder eine Verbindung der Formel ZRZ', wobei Z und Z' unabhängig Abgangsgruppen sind und R eine gerade Kette oder eine verzweigte Alkylengruppe ist;
gegenüber einer Additionsreaktion zum Erlangen eines Additionsprodukts; und
(b1) Reagieren des Additionsprodukts von Epichlorhydrin mit einer Verbindung der folgenden Formel (XI): 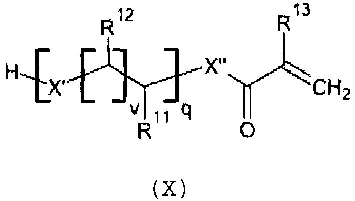 wobei
R¹¹ und R¹² die gleich oder unterschiedlich sein können, unabhängig ein Wasserstoffatom oder eine gerade Kette oder eine verzweigte C₁₋₆-Alkylgruppe darstellen,
R¹³ ein Wasserstoffatom oder eine gerade Kette oder eine verzweigte C₁₋₆-Alkylgruppe darstellt, die durch eine Hydroxylgruppe oder Carboxylgruppe substituiert sein kann,
X' -NH-, ein Sauerstoffatom oder ein Schwefelatom ist,
X" -NH-, ein Sauerstoffatom oder ein Schwefelatom ist und
q unabhängig eine ganze Zahl von 0 bis 10 darstellt,
v unabhängig eine ganze Zahl von 1 bis 10 darstellt,
zum Erlangen einer polymerisierbaren Verbindung der Formel (I), (II) oder (III) nach Anspruch 1;
(b2) Reagieren des Additionsprodukts von Ethylencarbonat mit einer Verbindung der folgenden Formel (XI) 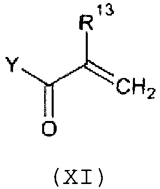 wobei Y eine Abgangsgruppe ist und R¹³ ein Wasserstoffatom oder eine gerade Kette oder eine verzweigte C₁₋₆-Alkylgruppe darstellt, oder wobei Y und R¹³ zusammen mit den Kohlenstoffatomen, an die sie gebunden sind, einen cyklischen Lactonring oder einen Carbonsäureanhydridring mit 4 bis 8 Kohlenstoffatomen bilden;
zum Erlangen einer polymerisierbaren Verbindung der Formel (I), (II) oder (III) nach Anspruch 1; oder
(b3) Reagieren des Additionsprodukts einer Verbindung der Formel ZRZ', wobei Z und Z' unabhängig Abgangsgruppen sind und R eine gerade Kette oder eine verzweigte Alkylengruppe ist, mit einer Verbindung der Formel (XI), wobei Y OH ist.

11. Verfahren zur Herstellung einer Dentalzusammensetzung nach Anspruch 1, umfassend das Unterwerfen eines Reaktionsgemischs, das eine oder mehrere Verbindungen der Formel (XII), (XIII) und/oder Formel (XIV) umfasst: 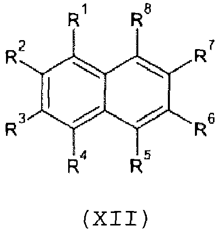 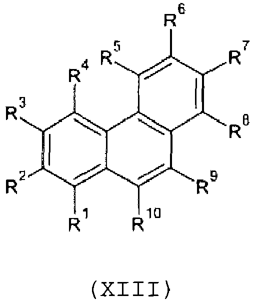 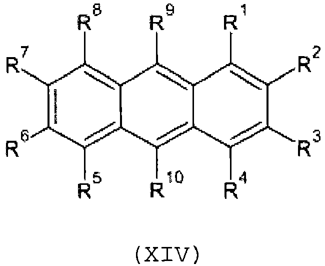 wobei
2 bis 4 Gruppen von R¹, R², R³, R⁴, R⁵, R⁶, R⁷ und R⁸ und, falls vorhanden, R⁹ und R¹⁰ unabhängig eine Gruppe der folgenden Formel (XV) sind: 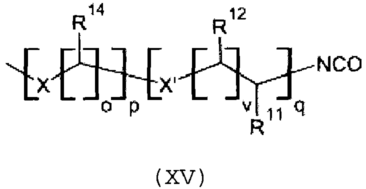 wobei
X -NH-, ein Sauerstoffatom oder ein Schwefelatom ist,
X' -NH-, ein Sauerstoffatom oder ein Schwefelatom ist,
R¹¹ und R¹²
die gleich oder unterschiedlich sein können, unabhängig ein Wasserstoffatom oder eine gerade Kette oder eine verzweigte C₁₋₆-Alkylgruppe darstellen,
R¹⁴ das gleich oder unterschiedlich sein kann, unabhängig ein Wasserstoffatom, eine Hydroxylgruppe oder eine gerade Kette oder eine verzweigte C₁₋₆-Alkylgruppe darstellt,
o unabhängig eine ganze Zahl von 2 bis 10 darstellt,
p unabhängig eine ganze Zahl von 0, 1 oder 2 ist;
q unabhängig eine ganze Zahl von 0 bis 10 darstellt;
v unabhängig eine ganze Zahl von 1 bis 10
darstellt, und die verbleibenden Gruppen von R¹, R², R³, R⁴, R⁵, R⁶, R⁷ und R⁸ und, falls vorhanden, R⁹ und R¹⁰ unabhängig ein Wasserstoffatom, ein Halogenatom, eine gerade Kette oder eine verzweigte C₁₋₆-Alkylgruppe, eine C₃₋₆-Cycloalkylgruppe oder eine C₁₋₆Alkyl-C₃₋₆Cycloalkylgruppe darstellen, wobei die Gruppen 1 bis 3 Heteroatome enthalten können, die aus Stickstoff-, Sauerstoff- und Schwefelatomen ausgewählt oder durch 1 bis 3 Substituenten substituiert sind, die ausgewählt sind aus Halogenatomen oder Hydroxygruppen, gegenüber einer Additionsreaktion mit einer Verbindung der folgenden Formel (XVI): 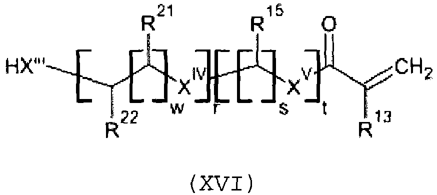 wobei
X"' -NH-, ein Sauerstoffatom oder ein Schwefelatom ist,
X^{IV} -NH-, ein Sauerstoffatom oder ein Schwefelatom ist,
X^{V} -NH-, ein Sauerstoffatom oder ein Schwefelatom ist,
R²¹ und R²²
die gleich oder unterschiedlich sein können, unabhängig ein Wasserstoffatom oder eine gerade Kette oder eine verzweigte C₁₋₆-Alkylgruppe darstellen,
R¹³ ein Wasserstoffatom oder eine gerade Kette oder eine verzweigte C₁₋₆-Alkylgruppe darstellt, die durch eine Hydroxylgruppe oder Carboxylgruppe substituiert sein kann,
R¹⁵ das gleich oder unterschiedlich sein kann, unabhängig ein Wasserstoffatom, eine Hydroxylgruppe oder eine gerade Kette oder eine verzweigte C₁₋₆-Alkylgruppe darstellt,
s unabhängig eine ganze Zahl von 2 bis 10 darstellt,
t unabhängig eine ganze Zahl von 0, 1 oder 2 ist;
r das gleich oder unterschiedlich sein kann, unabhängig eine ganze Zahl von 0 bis 10 darstellt;
w unabhängig eine ganze Zahl von 1 bis 10 darstellt,
zum Erlangen einer polymerisierbaren Verbindung der Formel (I), (II) oder (III) nach Anspruch 1.

12. Verfahren zur Herstellung einer Dentalzusammensetzung nach Anspruch 1, umfassend das Unterwerfen eines Reaktionsgemischs, das eine oder mehrere Verbindungen der Formel (XVII), (XVIII) und/oder Formel (XIX) umfasst: 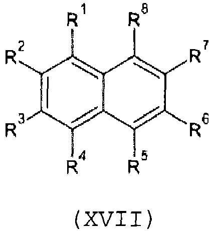 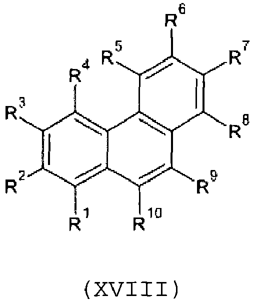 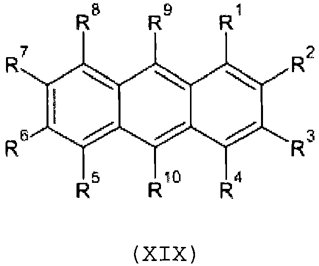 wobei
2 bis 4 Gruppen von R¹, R², R³, R⁴, R⁵, R⁶, R⁷ und R⁸ und, falls vorhanden, R⁹ und R¹⁰ Isocyanatomethylgruppen sind und die verbleibenden Gruppen R¹, R², R³, R⁴, R⁵, R⁶, R⁷ und R⁸ und, falls vorhanden, R⁹ und R¹⁰ unabhängig ein Wasserstoffatom, ein Halogenatom, eine gerade Kette oder eine verzweigte C₁₋₆-Alkylgruppe, eine C₃₋₆-Cycloalkylgruppe oder eine C₁₋₆-Alkyl-C₃₋₆-Cycloalkylgruppe darstellen, wobei die Gruppen 1 bis 3 Heteroatome enthalten können, die aus Stickstoff-, Sauerstoff- und Schwefelatomen ausgewählt oder durch 1 bis 3 Substituenten substituiert sind, die aus Halogenatomen oder Hydroxygruppen ausgewählt sind, gegenüber einer Additionsreaktion mit einer Verbindung der folgenden Formel (XX): 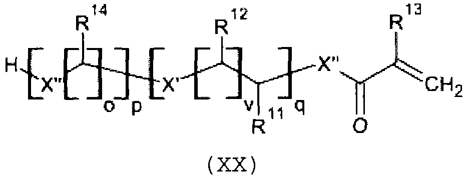 wobei
X' -NH-, ein Sauerstoffatom oder ein Schwefelatom ist,
X" -NH-, ein Sauerstoffatom oder ein Schwefelatom ist,
X"' -NH-, ein Sauerstoffatom oder ein Schwefelatom ist,
R¹¹ und R¹²
die gleich oder unterschiedlich sein können, unabhängig ein Wasserstoffatom oder eine gerade Kette oder eine verzweigte C₁₋₆-Alkylgruppe darstellen,
R¹³ ein Wasserstoffatom oder eine gerade Kette oder eine verzweigte C₁₋₆-Alkylgruppe darstellt, die durch eine Hydroxylgruppe oder Carboxylgruppe substituiert sein kann,
R¹⁴ das gleich oder unterschiedlich sein kann, unabhängig ein Wasserstoffatom, eine Hydroxylgruppe oder eine gerade Kette oder eine verzweigte C₁₋₆-Alkylgruppe darstellt,
o das gleich oder unterschiedlich sein kann, unabhängig eine ganze Zahl von 2 bis 10 darstellt,
p das gleich oder unterschiedlich sein kann, unabhängig eine ganze Zahl von 0, 1 oder 2 darstellt;
q das gleich oder unterschiedlich sein kann, unabhängig eine ganze Zahl von 0 bis 10 darstellt;
v unabhängig eine ganze Zahl von 1 bis 10 darstellt,
zum Erlangen einer polymerisierbaren Verbindung der Formel (I), (II) oder (III) nach Anspruch 1.

13. Verfahren nach einem der Ansprüche 10 bis 12, das ferner einen Schritt des Reagierens einer polymerisierbaren Verbindung der Formel (I), (II) oder (III) nach Anspruch 1, die Hydroxylgruppen in irgendwelchen von R¹³, R¹⁴ und/oder R¹⁵ enthält, mit einer Isocyanatverbindung, einer Diisocyanatverbindung, Isothiocyanatverbindung, einer Carbonsäurehalogenidverbindung, einem cyklischen Carbonsäureanhydrid oder einer Carbonsäureverbindung zum Herstellen einer substituierten Hydroxylgruppe umfasst.

14. Dentalzusammensetzung, die durch das Verfahren nach einem der Ansprüche 10 bis 13 erlangbar ist.

15. Verwendung einer Dentalzusammensetzung nach Anspruch 14 zur Herstellung einer Dentalzusammensetzung wie definiert nach einem der Ansprüche 8 bis 10.

## Revendications

1. Composition dentaire comprenant un composé polymérisable de la formule suivante (I), (II) et/ou de la formule (III) : 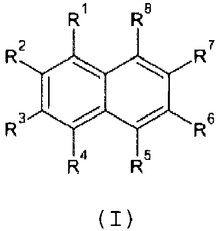 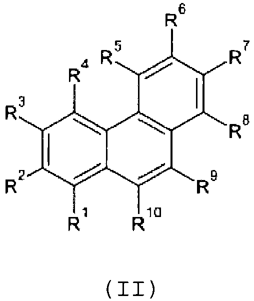 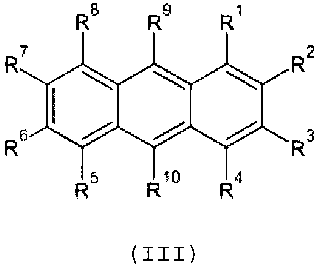 dans laquelle
2 à 4 groupes de R¹, R², R³, R⁴, R⁵, R⁶, R⁷ et R⁸, et, si présent, R⁹, et R¹⁰, qui sont indépendants l'un de l'autre, représentent un groupe de la formule suivante (IV), (V), ou (VI): 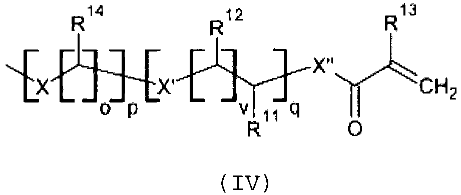 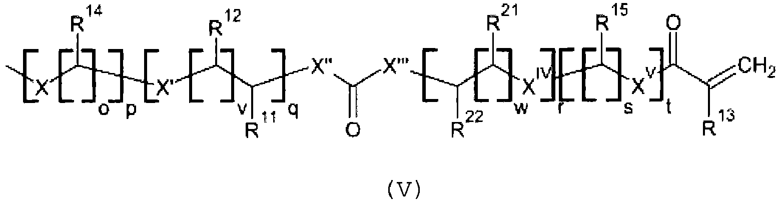 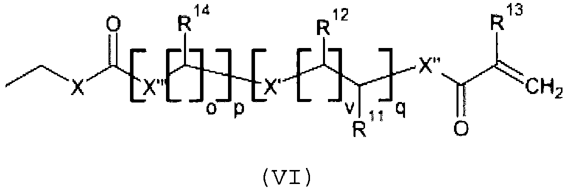 dans laquelle
X est -NH-, un atome d'oxygène ou un atome de soufre,
X' est -NH-, un atome d'oxygène ou un atome de soufre,
X" est -NH-, un atome d'oxygène ou un atome de soufre,
X'" est -NH-, un atome d'oxygène ou un atome de soufre,
X^{IV} est -NH-, un atome d'oxygène ou un atome de soufre,
X^{V} est -NH-, un atome d'oxygène ou un atome de soufre,
R¹¹, R¹², R²¹ et R²²
qui peuvent être identiques ou différents, représentent indépendamment un atome d'hydrogène ou un groupe alkyle à chaîne droite ou ramifié C₁₋₆,
R¹³ représente un atome d'hydrogène ou un groupe alkyle à chaîne droite ou ramifié C₁₋₆ qui peut être substitué par un groupe hydroxyle ou un groupe carboxyle,
R¹⁴ et R¹⁵
qui peuvent être identiques ou différents, représentent indépendamment un atome d'hydrogène, un groupe hydroxyle qui peut être substitué ou un groupe alkyle à chaîne droite ou ramifié C₁₋₆,
o et s qui peuvent être identiques ou différents, représentent indépendamment un nombre entier allant de 2 à 10,
p et t qui peuvent être identiques ou différents, représentent indépendamment un nombre entier allant de 0, 1 ou 2 ;
q et r qui peuvent être identiques ou différents, représentent indépendamment un nombre entier allant de 0 à 10 ;
v et w qui peuvent être identiques ou différents, représentent indépendamment un nombre entier allant de 1 à 10
et les groupes restants de R¹, R², R³, R⁴, R⁵, R⁶, R⁷, et R⁸, et, si présent, R⁹, et R¹⁰ représentent indépendamment un atome d'hydrogène, un atome d'halogène, un groupe alkyle à chaîne droite ou ramifié C₁₋₆, un groupe cycloalkyle en C₃₋₆, ou un groupe alkyle en C₁₋₆ cycloalkyle en C₃₋₆, ces groupes peuvent contenir 1 à 3 hétéroatomes sélectionnés parmi l'azote, l'oxygène et des atomes de soufre ou être substitués par 1 à 3 substituants sélectionnés parmi des atomes d'halogène ou de groupes hydroxy.

2. Composition dentaire selon la revendication 1, qui comprend un composé de la formule (I).

3. Composition dentaire selon la revendication 1, dans laquelle 2 groupes de R¹, R², R³, R⁴, R⁵, R⁶, R⁷, et R⁸, et, si présent, R⁹, et R¹⁰, représentent un groupe de la formule (IV).

4. Composition dentaire selon la revendication 3, dans laquelle les groupes restants de R¹, R², R³, R⁴, R⁵, R⁶, R⁷, et R⁸, et, si présent, R⁹, et R¹⁰ représentent un atome d'hydrogène.

5. Composition dentaire selon l'une quelconque des revendications précédentes, dans laquelle R² et R⁷, qui peuvent être identiques ou différents, représentent un groupe de la formule (IV).

6. Composition dentaire selon la revendication 4, dans laquelle R¹, R³, R⁴, R⁵, R⁶ et R⁸ représentent un atome d'hydrogène.

7. Composition dentaire selon la revendication 1, qui comprend un composé de formule (II).

8. Composition dentaire selon l'une quelconque des revendications précédentes, qui comprend en outre un remplisseur et/ou un solvant et qui comprend de préférence en outre un système initiateur de polymérisation.

9. Composition dentaire selon l'une quelconque des revendications précédentes, laquelle est sélectionnée parmi une composition dentaire adhésive, un scellement pour puits et fissures dentaires, un composite dentaire et une composition de remplissage de canal radiculaire.

10. Procédé pour la préparation d'une composition dentaire selon la revendication 1, comprenant les étapes de
(a) la soumission d'un mélange réactionnel comprenant
(a1) un ou plusieurs composés de formule (VII), (VIII) et/ou de la formule (IX) : 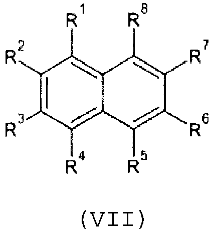 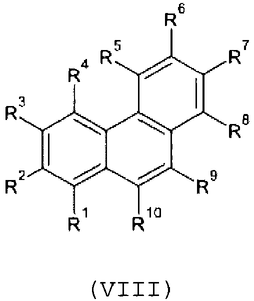 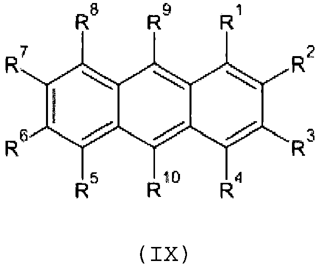 dans laquelle
2 à 4 groupes de R¹, R², R³, R⁴, R⁵, R⁶, R⁷, et R⁸, et, si présent, R⁹, et R¹⁰ sont des groupes -XH dans laquelle X est -NH-, un atome d'oxygène ou un atome de soufre, et les groupes restants de R¹, R², R³, R⁴, R⁵, R⁶, R⁷, et R⁸, et, si présent, R⁹, et R¹⁰ représentent indépendamment un atome d'hydrogène, un atome d'halogène, un groupe alkyle à chaîne droite ou ramifié C₁₋₆, un groupe cycloalkyle C₃₋₆, ou un groupe alkyle C₁₋₆ cycloalkyle C₃₋₆, ces groupes peuvent contenir 1 à 3 hétéroatomes sélectionnés parmi l'azote, l'oxygène et des atomes de soufre ou être substitués par 1 à 3 substituants sélectionnés parmi des atomes d'halogène ou des groupes hydroxy, et
(a2) l'épichlorohydrine, le carbonate d'éthylène ou un composé de la formule ZRZ', dans laquelle Z et Z' sont indépendamment des groupes partants et R est un groupe alkylène à chaîne droite ou ramifié ;
à une réaction d'addition pour l'obtention d'un produit d'addition ; et
(b1) la réaction du produit d'addition d'épichlorohydrine avec un composé de la formule suivante (XI) : 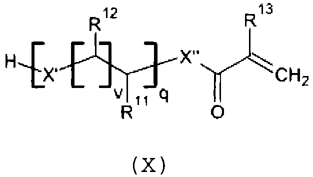 dans laquelle
R¹¹ et R¹²
qui peuvent être identiques ou différents, représentent indépendamment un atome d'hydrogène ou un groupe alkyle à chaîne droite ou ramifié C₁₋₆,
R¹³ représente un atome d'hydrogène ou un groupe alkyle à chaîne droite ou ramifié C₁₋₆ qui peut être substitué par un groupe hydroxyle ou groupe carboxyle,
X' est -NH-, un atome d'oxygène ou un atome de soufre,
X" est -NH-, un atome d'oxygène ou un atome de soufre, et
q représente indépendamment un nombre entier allant de 0 à 10,
v représente indépendamment un nombre entier allant de 1 à 10,
pour l'obtention d'un composé polymérisable de formule (I), (II) ou (III) selon la revendication 1 ;
(b2) la réaction du produit d'addition du carbonate d'éthylène avec un composé de la formule suivante (XI) 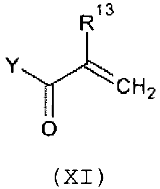 dans laquelle Y est un groupe partant et R¹³ représente un atome d'hydrogène ou un groupe alkyle à chaîne droite ou ramifié C₁₋₆, ou dans laquelle Y et R¹³ forment ensemble avec les atomes de carbone auxquels ils sont liés à un noyau de lactone cyclique ou un noyau d'anhydride d'acide carboxylique ayant 4 à 8 atomes de carbone ;
pour l'obtention d'un composé polymérisable de formule (I), (II) ou (III) selon la revendication 1 ; ou
(b3) la réaction du produit d'addition d'un composé de la formule ZRZ', dans laquelle Z et Z' sont indépendamment des groupes partants et R est un groupe alkylène à chaîne droite ou ramifié, avec un composé de formule (XI) dans laquelle Y est OH.

11. Procédé pour la préparation d'une composition dentaire selon la revendication 1, comprenant la soumission d'un mélange réactionnel comprenant un ou plusieurs composés de formule (XII), (XIII) et/ou de la formule (XIV) : 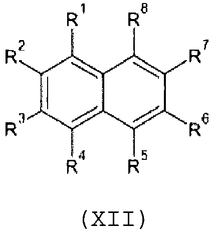 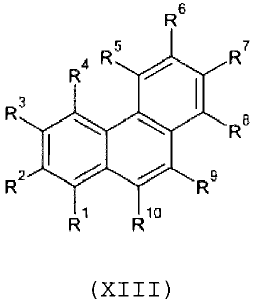 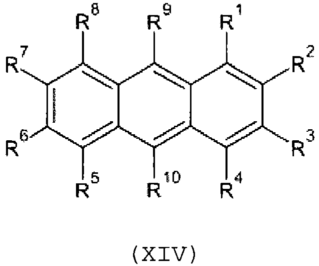 dans laquelle
2 à 4 groupes de R¹, R², R³, R⁴, R⁵, R⁶, R⁷, et R⁸, et, si présent, R⁹, et R¹⁰ sont indépendamment un groupe de la formule suivante (XV): 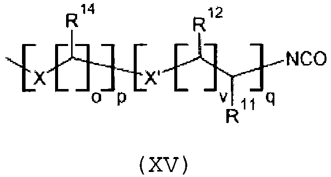 dans laquelle
X est -NH-, un atome d'oxygène ou un atome de soufre,
X' est -NH-, un atome d'oxygène ou un atome de soufre,
R¹¹ et R¹²
qui peuvent être identiques ou différents, représentent indépendamment un atome d'hydrogène ou un groupe alkyle à chaîne droite ou ramifié C₁₋₆,
R¹⁴ qui peuvent être identiques ou différents, représentent indépendamment un atome d'hydrogène, un groupe hydroxyle, ou un groupe alkyle à chaîne droite ou ramifié C₁₋₆,
ο représentent indépendamment un nombre entier allant de 2 à 10,
p indépendamment est un nombre entier allant de 0, 1 ou 2 ;
q représentent indépendamment un nombre entier allant de 0 à 10 ;
v représente indépendamment un nombre entier allant de 1 à 10,
et les groupes restants de R¹, R², R³, R⁴, R⁵, R⁶, R⁷, et R⁸, et, si présent, R⁹, et R¹⁰ représentent indépendamment un atome d'hydrogène, un atome d'halogène, un groupe alkyle à chaîne droite ou ramifié C₁₋₆, un groupe cycloalkyle en C₃₋₆, ou un groupe alkyle en C₁₋₆ cycloalkyle en C₃₋₆, ces groupes peuvent contenir 1 à 3 hétéroatomes sélectionnés parmi l'azote, l'oxygène, et des atomes de soufre ou être substitués par 1 à 3 substituants sélectionnés parmi des atomes d'halogène ou groupes hydroxy, à une réaction d'addition avec un composé de la formule suivante (XVI): 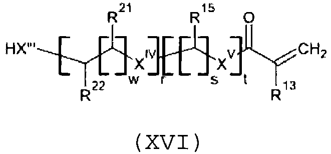 dans laquelle
X"' est -NH-, un atome d'oxygène ou un atome de soufre,
X^{IV} est -NH-, un atome d'oxygène ou un atome de soufre,
X^{V} est -NH-, un atome d'oxygène ou un atome de soufre,
R²¹ et R²²
qui peuvent être identiques ou différents, représentent indépendamment un atome d'hydrogène ou un groupe alkyle à chaîne droite ou ramifié C₁₋₆,
R¹³ représente un atome d'hydrogène ou un groupe alkyle à chaîne droite ou ramifié C₁₋₆ qui peut être substitué par un groupe hydroxyle ou un groupe carboxyle,
R¹⁵ qui peuvent être identiques ou différents, représentent indépendamment un atome d'hydrogène, un groupe hydroxyle, ou un groupe alkyle à chaîne droite ou ramifié C₁₋₆,
s représentent indépendamment un nombre entier allant de 2 à 10,
t indépendamment est un nombre entier allant de 0, 1 ou 2 ;
r qui peuvent être identiques ou différents, représentent indépendamment un nombre entier allant de 0 à 10 ;
w représente indépendamment un nombre entier allant de 1 à 10,
pour l'obtention d'un composé polymérisable de formule (I), (II) ou (III) selon la revendication 1.

12. Procédé pour la préparation d'une composition dentaire selon la revendication 1, comprenant la soumission d'un mélange réactionnel comprenant un ou plusieurs composés de formule (XVII), (XVIII) et/ou de la formule (XIX): 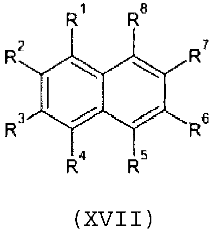 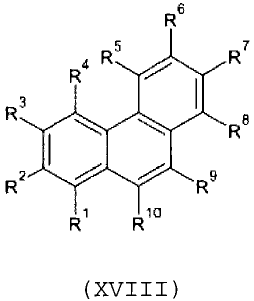 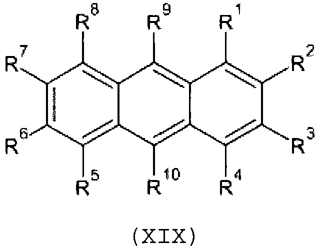 dans laquelle
2 à 4 groupes de R¹, R², R³, R⁴, R⁵, R⁶, R⁷, et R⁸, et, si présent, R⁹, et R¹⁰ sont des groupes isocyanatométhyle, et les groupes restants de R¹, R², R³, R⁴, R⁵, R⁶, R⁷, et R⁸, et, si présent, R⁹, et R¹⁰ représentent indépendamment un atome d'hydrogène, un atome d'halogène, un groupe alkyle à chaîne droite ou ramifié C₁₋₆, un groupe cycloalkyle C₃₋₆, ou un groupe alkyle en C₁₋₆ cycloalkyle en C₃₋₆, ces groupes peuvent contenir 1 à 3 hétéroatomes sélectionnés parmi l'azote, l'oxygène et des atomes de soufre ou peuvent être substitués par 1 à 3 substituants sélectionnés parmi des atomes d'halogène ou groupes hydroxy, à une réaction d'addition avec un composé de la formule suivante (XX): 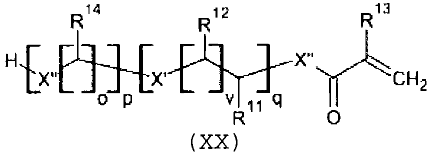 dans laquelle
X' est -NH-, un atome d'oxygène ou un atome de soufre,
X" est -NH-, un atome d'oxygène ou un atome de soufre,
X'" est -NH-, un atome d'oxygène ou un atome de soufre,
R¹¹ et R¹²
qui peuvent être identiques ou différents, représentent indépendamment un atome d'hydrogène ou un groupe alkyle à chaîne droite ou ramifié C₁₋₆,
R¹³ représente un atome d'hydrogène ou un groupe alkyle à chaîne droite ou ramifié C₁₋₆ qui peut être substitué par un groupe hydroxyle ou un groupe carboxyle,
R¹⁴ qui peuvent être identiques ou différents, représentent indépendamment un atome d'hydrogène, un groupe hydroxyle, ou un groupe alkyle à chaîne droite ou ramifié C₁₋₆,
o qui peuvent être identiques ou différents, représentent indépendamment un nombre entier allant de 2 à 10,
p qui peuvent être identiques ou différents, indépendamment est un nombre entier allant de 0, 1 ou 2 ; q qui peuvent être identiques ou différents, représentent indépendamment un nombre entier allant de 0 à 10 ;
v représente indépendamment un nombre entier allant de 1 à 10,
pour l'obtention d'un composé polymérisable de formule (I), (II) ou (III) selon la revendication 1.

13. Procédé selon l'une quelconque des revendications 10 à 12, qui comprend en outre une étape de réaction d'un composé polymérisable de formule (I), (II) ou (III) selon la revendication 1, qui contient des groupes hydroxyle dans tout R¹³, R¹⁴, et/ou R¹⁵, avec un composé isocyanate, un composé diisocyanate, un composé isothiocyanate, un composé halogénure d'acide carboxylique, un composé anhydride d'acide carboxylique cyclique ou un composé d'acide carboxylique pour la préparation d'un groupe hydroxyle substitué.

14. Composition dentaire pouvant être obtenue par un procédé selon l'une quelconque des revendications 10 à 13.

15. Utilisation d'une composition dentaire selon la revendication 14 pour la préparation d'une composition dentaire selon l'une quelconque des revendications 8 à 10.
